# EUROPEAN PATENT APPLICATION

(11) **EP 4 707 289 A1**
(43) Date of publication of application: **11.03.2026**
(21) Application number: 24799887.5
(22) Date of filing: 29.04.2024
(51) Int. Cl.: C07J 41/00, A61K 31/713, A61K 9/127, A61P 35/00, A61P 31/00

(54) **STEROID-CATIONIC LIPID COMPOUND AND USE THEREOF**

(30) Priority: 29.04.2023 CN 202310482073
(71) Applicant: Cansino (Shanghai) Biological Research Co., Ltd., Shanghai 201203 (CN); Jenkem Technology Co. Ltd. (Tianjin), Tianjin 300462 (CN)
(72) Inventor: WANG, Zhenghua, Shanghai 201203 (CN); HAO, Jing, Tianjin 300462 (CN); WANG, Haomeng, Shanghai 201203 (CN); YAN, Shengyong, Tianjin 300462 (CN); YAN, Zhihong, Shanghai 201203 (CN); XIONG, Yanli, Tianjin 300462 (CN); DAI, Shouyi, Shanghai 201203 (CN); LIU, Jian, Shanghai 201203 (CN); GUO, Jun, Tianjin 300462 (CN); QIU, Dongxu, Shanghai 201203 (CN); WANG, Qingbin, Tianjin 300462 (CN); ZHU, Tao, Shanghai 201203 (CN); ZHAO, Xuan, Tianjin 300462 (CN); YU, Xuefeng, Shanghai 201203 (CN)
(74) Representative: Finnegan Europe LLP
(86) International application number: PCT/CN2024/090628
(87) International publication number: WO 2024/227430

(57) **Abstract**

The present invention provides a steroid-cationic lipid compound having a structure represented by formula (I), and use thereof. The compound can be used in the preparation of lipid nanoparticles (LNPs) for delivering a therapeutic agent and/or a prophylactic agent. The LNPs prepared by using the steroid-cationic lipid compound of the present invention have good stability and transfection efficiency. The LNPs are used for delivering nucleic acid, such as mRNA, and can efficiently and stably deliver a bioactive substance to a target cell or organ. In addition, the LNPs have high stability, can be used for developing a freeze-dried preparation of mRNA, can also be used for aerosol inhalation administration of mRNA, and can cause a high specific antibody response in an experimental animal body, and the compound is safer.

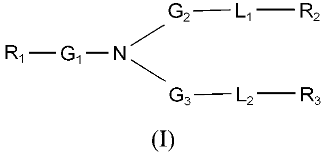

## Description

### TECHNICAL FIELD

The present disclosure pertains to the field of biopharmaceuticals, and particularly relates to a steroid-cationic lipid compound and use thereof in the delivery of a bioactive substance.

### BACKGROUND

Nucleic acid drugs primarily refer to compounds containing nucleotide or deoxynucleotide structures with genetic characteristics and pharmacological activity, which can be used for treating tumors, tissue regeneration, wound healing, pulmonary fibrosis, inflammatory diseases, microbial infections, etc. After being injected into the human body, the nucleic acid drugs require an efficient and safe drug delivery system to deliver them to lesion sites. This drug delivery system needs to remain in the body for a sufficient duration to accurately target the lesion sites while avoiding damage to normal cells. Currently, drug delivery systems may be categorized into viral carriers and non-viral carriers. Viral carriers have relatively fewer applications in nucleic acid drugs due to their immunogenicity, tumorigenicity, and limited drug loading; non-viral carriers, such as polymers and lipids (liposomes or LNPs), can bind nucleic acid drugs to specific ligands to enable them to target specific cells, and have relatively more applications in current nucleic acid drugs. At present, LNPs are one of the most commonly used delivery systems for nucleic acid drug research, and LNP delivery systems can safely and effectively deliver nucleic acids. They have the advantages of high nucleic acid encapsulation efficiency, capability of effective cell transfection, strong tissue penetration, low cytotoxicity and immunogenicity, and the like, and are highly advantageous compared with other drug delivery systems. Therefore, LNP delivery systems have broad development and application prospects.

In the prior art, LNP delivery systems are often made from ingredients such as ionizable lipids (cationic lipids), steroids, neutral lipids, polyethylene glycol lipids, nucleic acid drugs, and the like. For example, the patent document AU2020325221A1 discloses a composition of target cell delivery LNPs, which comprises: (i) an ionizable lipid; (ii) a sterol or other structural lipids; (iii) a non-cationic helper lipid or phospholipid; (iv) a PEG lipid; and (v) an agent (e.g., a nucleic acid molecule) encapsulated in the LNP, where the four components are assembled in a specific ratio to enhance the efficiency of delivery to target cells. The patent document WO2021/250263 A1 discloses a composition comprising an ionizable lipid, a phospholipid, a sterol, a polyethylene glycol lipid, and one or more nucleic acids, and discloses a composition comprising less than about 1 mol% of a C14-PEG2000 lipid and a specific percentage of other lipids. The patent CN102712935B discloses a lipid particle comprising: a cationic lipid; a neutral lipid, a zwitterionic lipid, or an anionic lipid; a polyethylene glycol lipid; a sterol; and a nucleic acid, where the above components are assembled into a lipid particle having a solid core, and the solid core can achieve higher coating efficiency. The patent document WO 2021/055849A1 discloses a lipid having the following structure: 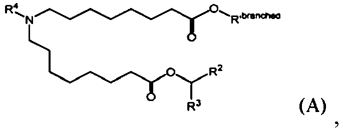 and the structure can improve its safety, effectiveness, and specificity. The patent document WO2021/026358Al discloses a target cell delivery lipid nanoparticle (LNP), comprising: (i) an ionizable lipid; (ii) a sterol or other structural lipid; (iii) a non-cationic helper lipid or phospholipid; (iv) a payload; and (v) a polyethylene glycol lipid, which is used as a drug delivery system to allow for safety and effectiveness.In recent years, it has been found that ionizable lipid compounds with the introduction of cholesterol can also be used in nucleic acid drug delivery. The patent document US7514099B2 discloses a cholesterol amino lipid compound CLinDMA 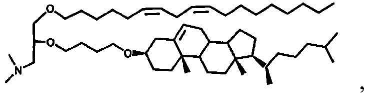 which can form a four-component LNP with a phospholipid, cholesterol, and a polyethylene glycol lipid, or form a five-component LNP with a phospholipid, a DMOBA lipid, cholesterol, and a PEG lipid to deliver an siRNA. The patent document CN112424214A discloses an ionizable cationic lipid compound (3) 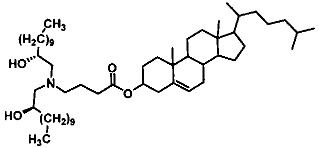 formed from cholesterol and a linear olefin, which forms a lipid nanoparticle with cholesterol, DPPC, DOPE, and DMG-PEG200 to deliver a nucleic acid. The above two compounds both require 4 or even 5 different lipid excipients to form a nucleic acid drug delivery carrier formulation, which is complicated to construct. In order to adapt to different application scenarios, it is also particularly important to develop different delivery systems suitable for different routes of administration, such as the use of aerosol inhalation administration in treating lung-related diseases or preventing infections caused by respiratory tract-related pathogens. At present, the stable storage of mRNA-LNPs remains a major difficulty, and mRNA-LNPs can be stably stored at 2-8 °C or even at room temperature by lyophilization. It can be concluded that, in order to further obtain LNP delivery systems that are safer, more efficient, and more stable, have simple construction, and can be suitable for use in different routes of administration simultaneously, their components and the structures of the components need to be further developed, by optimizing the components and optimizing the cationic lipids in the composition in the prior art.

### SUMMARY

In a first aspect, the present disclosure provides a steroid-cationic lipid compound having a structure represented by formula (I): 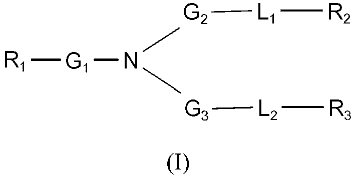
wherein R₁ is selected from -OR₄, -NR₄R₅, -NR₄C(=O)R₅, -C(=O)OR₅, -OC(=O)R₅, -OC(=O)OR₅, - CN, a nitrogen-containing heterocyclic group, and guanidino;
R₄ and R₅ are each independently selected from H, C₁₋₉ alkyl, C₂₋₉ alkenyl, C₂₋₉ alkynyl, C₃₋₈ cycloalkyl, C₃₋₈ cycloalkenyl, and C₃₋₈ cycloalkynyl;
G₁ is selected from a chemical bond (-), C₁₋₉ alkylene, C₂₋₉ alkenylene, C₃₋₉ alkynylene, C₃₋₈ cycloalkylene, and C₃₋₈ cycloalkenylene;
R₁-G₁- has no more than 10 contiguous carbon atoms; for example, R₁-G₁- may be 1, 2, 3, 4, 5, 6, 7, 8, 9, or 10 contiguous carbon atoms.

G₂ and G₃ are each independently selected from a chemical bond (-), C₁₋₁₂ alkylene, C₂₋₁₂ alkenylene, C₂₋₁₂ alkynylene, C₃₋₁₂ cycloalkylene, C₃₋₁₂ cycloalkenylene, C₃₋₁₂ cycloalkynylene, and C₆₋₁₂ arylene; L₁ and L₂ are each independently selected from one or a combination of two or more of a chemical bond (-), -O-, -S-, -O(C=O)O-, -(C=O)NRₐ-, -NRₐ(C=O)-, -O(C=O)-, -(C=O)O-, -S-S-, -S(O)ₓ-, - OS(O)ₓO-, -C(=O)S-, -SC(=O)-, -NRₐC(=O)NR_{b}-, -OC(=O)NRₐ-, -NRₐC(=O)O-, -OC(=O)S-, - SC(=O)O-, -P(O)(ORₐ)O-, -OP(O)(ORₐ)O-, and C₁₋₁₂ alkylene;
wherein x is selected from 0, 1, and 2;
wherein Rₐ and R_{b} are each independently selected from H, C₁₋₁₂ alkyl, C₂₋₁₂ alkenyl, and C₂₋₁₂ alkyne; R₂ is selected from a naturally occurring or non-naturally occurring steroid;
R₃ is independently selected from a naturally occurring or non-naturally occurring steroid, C₆₋₂₄ alkyl, C₆₋₂₄ alkenyl, C₆₋₂₄ alkyne, and C₆₋₂₄ alkoxy.

Furthermore, R₁ is selected from -OR₄, -NR₄R₅, pyrazolyl, imidazolyl, piperazinyl, alkylpiperazine, piperidinyl, alkylpiperidine, guanidino, pyrrolyl, and pyrrolidinyl; R₄ and R₅ are each independently selected from H, C₁₋₉ alkyl, C₂₋₉ alkenyl, -C₂₋₉ alkynyl, C₃₋₈ cycloalkyl, and C₃₋₈ cycloalkenyl.

G₁ is a chemical bond (-), C₁₋₉ alkylene, C₂₋₉ alkenylene, C₃₋₈ cycloalkylene, or C₃₋₈ cycloalkenylene; preferably, G₁ is C₁₋₆ alkylene or C₂₋₆ alkenylene;
R₁-G₁- has no more than 10 contiguous carbon atoms; specifically, R₁-G₁- may have 1, 2, 3, 4, 5, 6, 7, 8, 9, or 10 contiguous carbon atoms;
G₂ and G₃ are each independently selected from a chemical bond (-), -C₁₋₁₂ alkylene, -C₂₋₁₂ alkenylene, and -C₂₋₁₂ alkynylene;
L₁ and L₂ are each independently selected from one or a combination of two or more of -O(C=O)-, - (C=O)O-, -S-S-, -O(C=O)O-, -NRₐC(=O)O-, -(C=O)NRₐ-, -NRₐ(C=O)-, -C(=O)S-, -SC(=O)-, - OC(=O)NRₐ-, and C₁₋₁₂ alkylene;
wherein Rₐ is H, C₁₋₁₂ alkyl, C₂₋₁₂ alkenyl, or C₂₋₁₂ alkyne;

Furthermore, R₁ is selected from -OR₄, -NR₄R₅, imidazolyl, piperazinyl, and guanidino;
R₄ and R₅ are each independently selected from H and C₁₋₅ alkyl;
G₁ is unsubstituted C₁₋₆ alkylene;
R₁-G₁- has no more than 10 contiguous carbon atoms; specifically, R₁-G₁- may have 1, 2, 3, 4, 5, 6, 7, 8, 9, or 10 contiguous carbon atoms;
G₂ and G₃ are each independently selected from a chemical bond (-) and C₁₋₁₀ alkylene;
L₁ and L₂ are each independently selected from -O-, -O(C=O)-, -(C=O)O-, -(C=O)S-, -O(C=O)O-, - NHC(=O)O-, -NHC(=O)-,

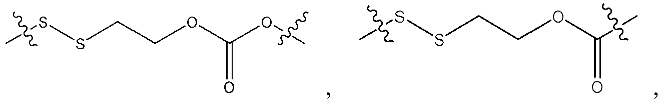

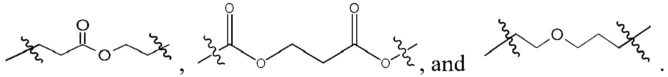

Further, R₂ is a sterol;
preferably, the sterol is a zoosterol or an oxidized or reduced form thereof; and/or the sterol is a phytosterol or an oxidized or reduced form thereof; and/or the sterol is a synthetic sterol or an oxidized or reduced form thereof;
more preferably, the sterol is selected from cholesterol, an oxidized form of cholesterol, a reduced form of cholesterol, alkyl lithocholate, stigmasterol, stigmastanol, campesterol, ergosterol, and sitosterol;
more preferably, the sterol is an oxidized form of cholesterol, a reduced form of cholesterol, alkyl lithocholate, stigmasterol, stigmastanol, campesterol, ergosterol, or sitosterol;
more preferably, the sterol is selected from avenasterol, β-sitosterol, brassicasterol, ergocalciferol, campesterol, cholestanol, cholesterol, coprosterol, dehydrocholesterol, desmosterol, dihydroergocalciferol, dihydrocholesterol, dihydroergosterol, dinosterol, epicholesterol, ergosterol, fucosterol, hexahydrolumisterol, hydroxycholesterol, lanosterol, lumisterol, saringosterol, sitostanol, sitosterol, stigmastanol, stigmasterol, cholic acid, glycocholic acid, taurocholic acid, deoxycholic acid, and lithocholic acid;
more preferably, the sterol has the following structural formula: 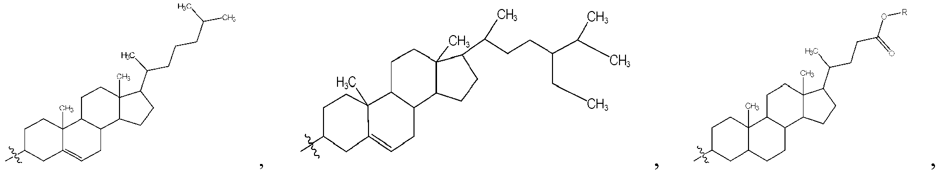 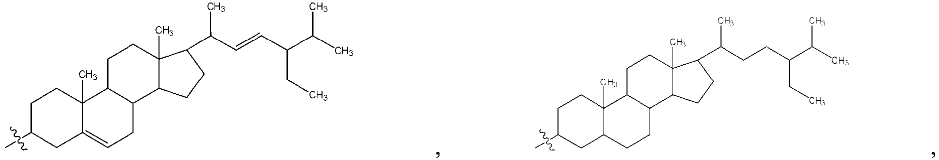 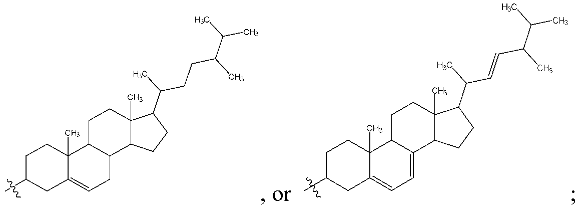
wherein R is C₁₋₂₀ alkyl.

Further, R₃ is selected from the following structures: 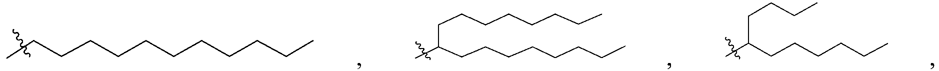 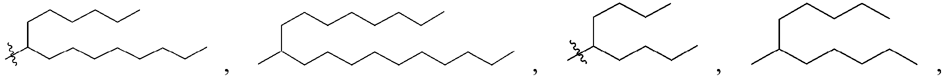 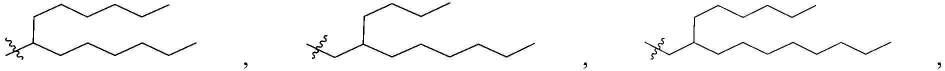 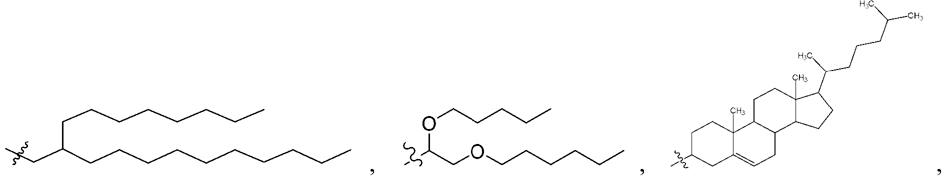 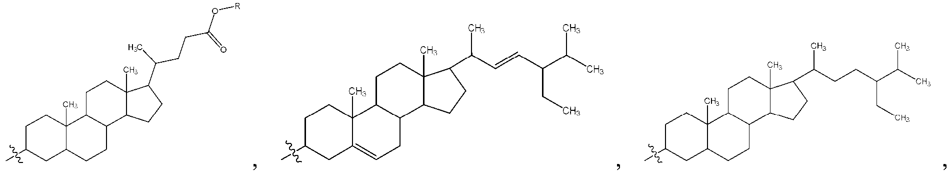 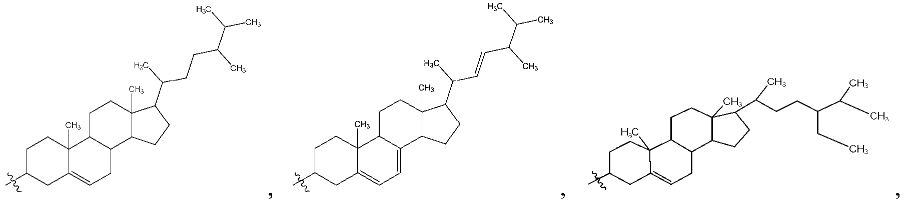 and

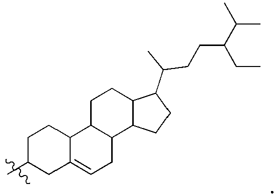

Preferably, R₃ is selected from the following structures:

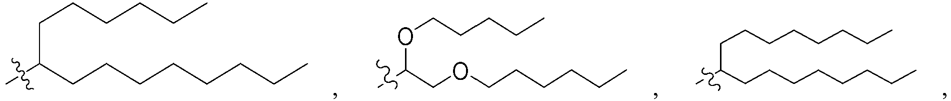

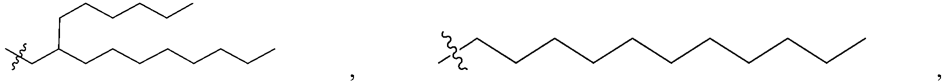

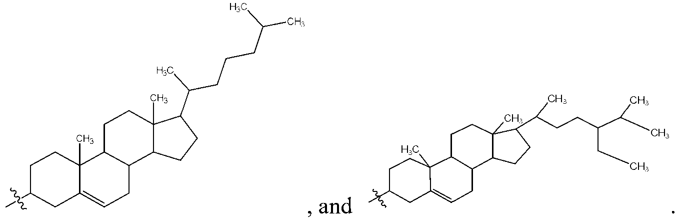

In one embodiment, the compound represented by formula (I) is selected from: 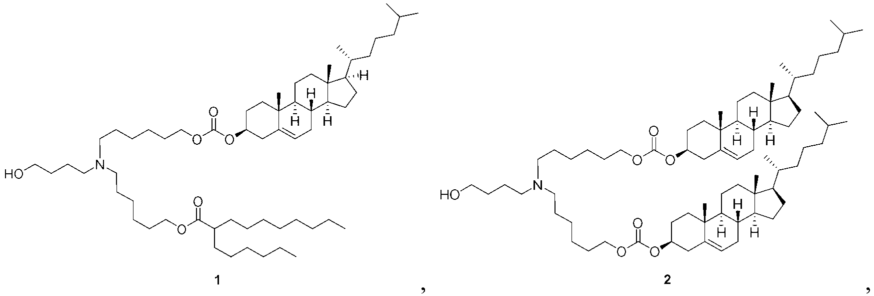 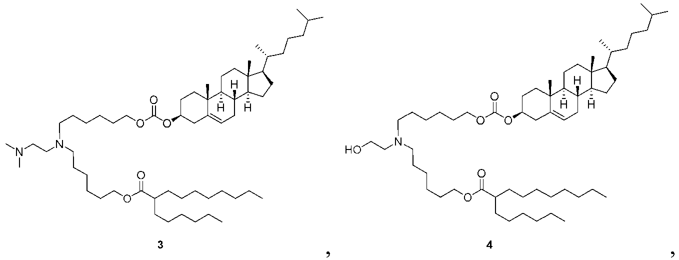 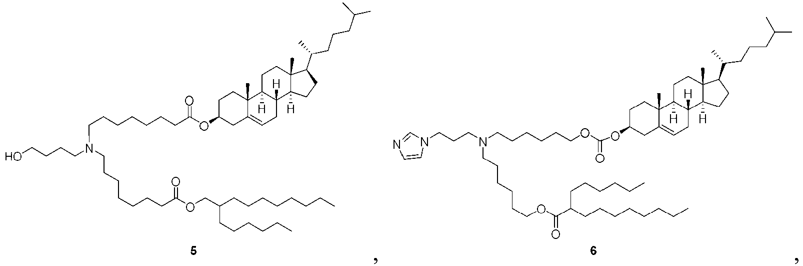 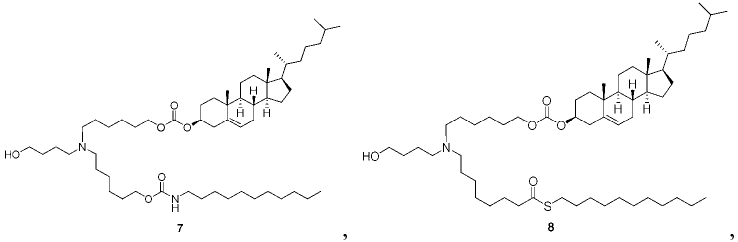 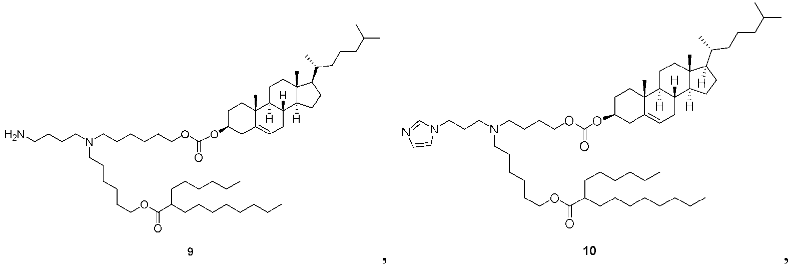 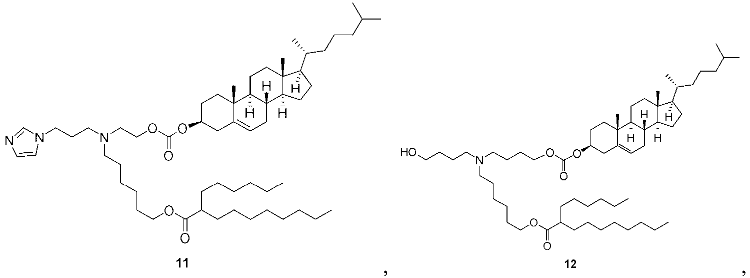 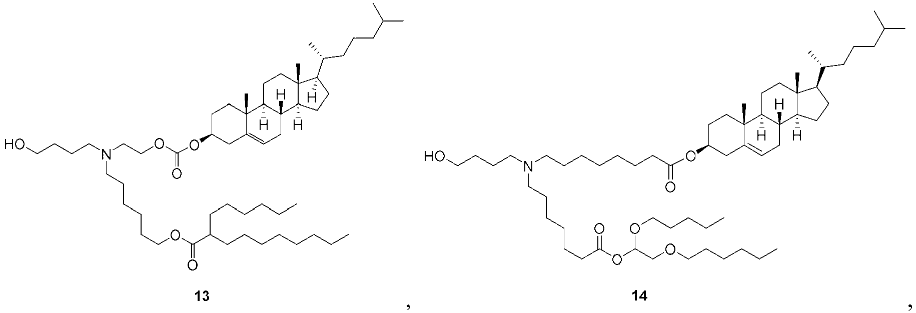 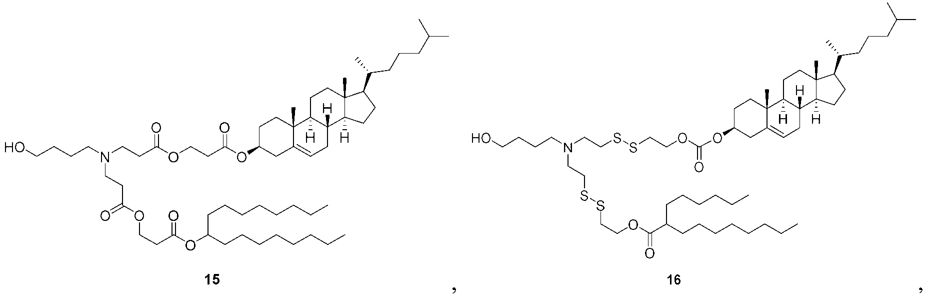 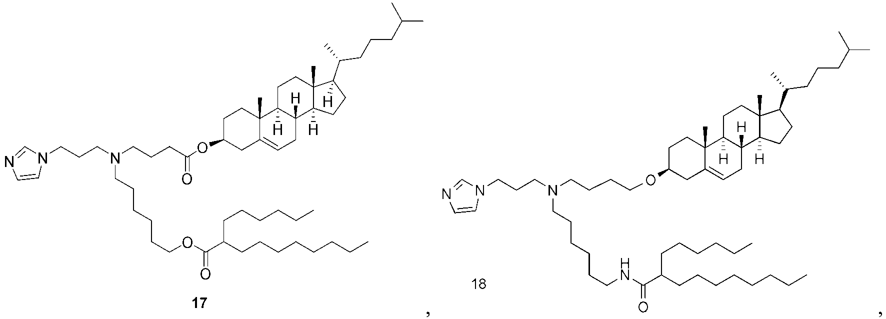 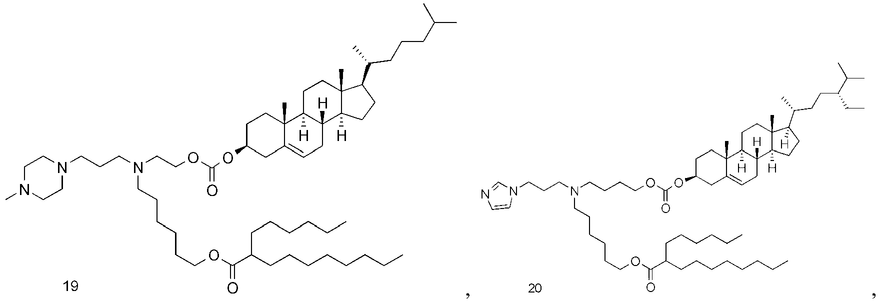 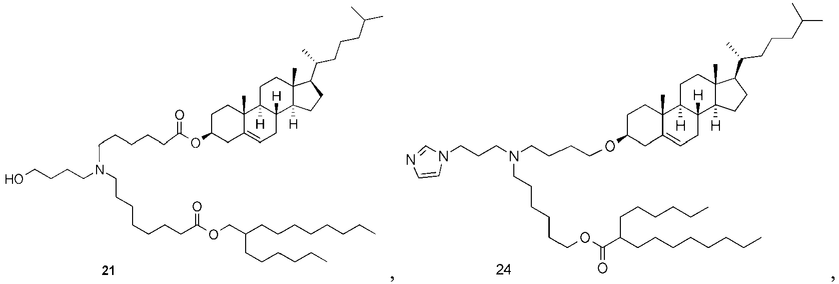 and

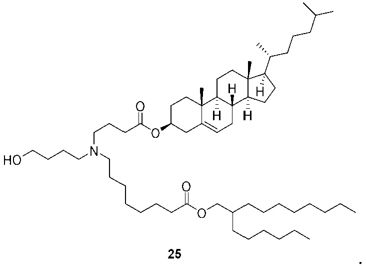

In another aspect, the present disclosure further provides a lipid nanoparticle comprising the aforementioned steroid-cationic lipid compound represented by formula (I).

In one embodiment, the lipid nanoparticle further comprises a polyethylene glycol lipid and at least one helper lipid; the helper lipid is selected from a neutral lipid, a zwitterionic lipid, and an anionic lipid.

In one embodiment, the polyethylene glycol lipid is selected from 2-[(polyethylene glycol)-2000]-N,N-ditetradecylacetamide (ALC-0159), 1,2-dimyristoyl-sn-glycero-methoxypolyethylene glycol (PEG-DMG), 1,2-distearoyl-sn-glycero-3-phosphoethanolamine-N-[amino(polyethylene glycol)] (PEG-DSPE), PEG-disterol glycerol (PEG-DSG), PEG-dipalmitoyl, PEG-dioleyl, PEG-distearyl, PEG-diacylglycerol amide (PEG-DAG), PEG-dipalmitoyl phosphatidylethanolamine (PEG-DPPE), and PEG-1,2-dimyristoyloxypropyl-3- (PEG-c-DMA); and/or,
the neutral lipid is selected from 1,2-distearoyl-sn-glycero-3-phosphocholine (DSPC), 1,2-dipalmitoyl-sn-glycero-3-phosphocholine (DPPC), 1,2-dioleoyl-sn-glycero-3-phosphoethanolamine (DOPE), 1,2-dipalmitoyl-sn-glycero-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), oleoylphosphatidylcholine (POPC), 1-palmitoyl-2-oleoylphosphatidylethanolamine (POPE), phosphocholine (DOPC), dimyristoylphosphatidylcholine (DMPC), phosphatidylcholine (PLPC), 1,2-distearoyl-sn-glycero-3-phosphocholine (DAPC), phosphatidylethanolamine (PE), egg phosphatidylcholine (EPC), dilauroylphosphatidylcholine (DLPC), dimyristoylphosphatidylcholine (DMPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1,2-diarachidoyl-sn-glycero-3-phosphocholine (DBPC), 1-stearoyl-2-palmitoylphosphatidylcholine (SPPC), 1,2-eicosenoyl-sn-glycero-3-phosphocholine (DEPC), lysophosphatidylcholine, dilinoleoylphosphatidylcholine distearoylphosphatidylethanolamine (DSPE), and lysophosphatidylethanolamine.

In one embodiment, in the lipid nanoparticle, the molar ratio of the steroid-cationic lipid represented by formula (I) to the helper lipid to the polyethylene glycol lipid is (20-80):(20-80):(0.5-20); preferably, in the lipid nanoparticle, the molar ratio of the steroid-cationic lipid represented by formula (I) to the helper lipid to the polyethylene glycol lipid is (30-80):(30-80):(0.5-20); more preferably, the molar ratio of the steroid-cationic lipid represented by formula (I) to the helper lipid to the PEG-lipid is (40-60):(40-60):(0.5-5); more preferably, the molar ratio of the steroid-cationic lipid represented by formula (I) to the helper lipid to the PEG-lipid is 49.25:49.25:1.5.

In one embodiment, the lipid nanoparticle has a diameter of 15-300 nm; preferably, the lipid nanoparticle has a diameter of 60-102 nm; more preferably, the lipid nanoparticle has a diameter of 80-90 nm.

In one embodiment, the encapsulation efficiency (%) of the lipid nanoparticle is 80%-100%.

In yet another aspect, the present disclosure further provides use of the aforementioned steroid-cationic lipid compound and the aforementioned lipid nanoparticle in preparing a bioactive substance delivery system.

In one embodiment, the bioactive substance may be a small molecule compound, a nucleic acid, an oligopeptide, etc.; preferably, the bioactive substance is a nucleic acid; more preferably, the bioactive substance is a DNA or an RNA; more preferably, the DNA includes a non-coding DNA (antisense DNA) or a coding DNA, and/or the RNA includes an antisense RNA, an saRNA, an mRNA, an lncRNA, an miRNA, an siRNA, a piRNA, a gRNA, a tsRNA, a circRNA, and a self-amplifying mRNA.

In one embodiment, the bioactive substance is used for preventing and/or treating cancer, inflammation, fibrotic diseases, autoimmune diseases, infection, psychiatric disorders, hematological diseases, chromosomal diseases, genetic diseases, connective tissue diseases, digestive diseases, ear-nose-throat diseases, endocrine diseases, eye diseases, reproductive diseases, heart diseases, kidney diseases, lung diseases, metabolic disorders, oral diseases, musculoskeletal diseases, neonatal screening, nutritional diseases, parasitic diseases, skin diseases, etc.

In one embodiment, the bioactive substance delivery system is an mRNA vaccine.

In one embodiment, the mRNA vaccine may be used for preventing a cancer, a virus infection, a bacterial infection, a fungal infection, etc. In one embodiment, the virus includes, but is not limited to, norovirus, Ebola virus, coronavirus (including novel coronavirus SARS-CoV-2), cytomegalovirus, Dengue virus, Zika virus, coxsackievirus, enterovirus, hepatitis virus, herpes simplex virus, human papilloma virus, influenza virus, Marburg virus, measles virus, poliovirus, rabies virus, rotavirus, measles virus, etc.

In yet another aspect, the present disclosure further provides a lipid nanoparticle comprising the aforementioned lipid nanoparticle and a bioactive substance.

In one embodiment, the bioactive substance may be a small molecule compound, a nucleic acid, an oligopeptide, etc.; preferably, the bioactive substance is a nucleic acid; more preferably, the bioactive substance is a DNA or an RNA; more preferably, the DNA includes a non-coding DNA (antisense DNA) or a coding DNA, and/or the RNA includes an antisense RNA, an saRNA, an mRNA, an lncRNA, an miRNA, an siRNA, a piRNA, a gRNA, a tsRNA, a circRNA, and a self-amplifying mRNA.

In one embodiment, a nitrogen-to-phosphorus ratio of the lipid nanoparticle is (1-15):1. For example, the nitrogen-to-phosphorus ratio of the lipid nanoparticle may be 1:1, 1.5:1, 2:1, 2.5:1, 3:1, 3.5:1, 4:1, 4.5:1, 5:1, 5.5:1, 6:1, 6.5:1, 7:1, 7.5:1, 8:1, 8.5:1, 9:1, 9.5:1, 10:1, 10.5:1, 11:1, 11.5:1, 12:1, 12.5:1, 13:1, 13.5:1, 14:1, 14.5:1, or 15:1; preferably, the nitrogen-to-phosphorus ratio of the lipid nanoparticle is (2-10):1; more preferably, the nitrogen-to-phosphorus ratio of the lipid nanoparticle is (4-8):1; more preferably, the nitrogen-to-phosphorus ratio of the composition is 6:1 or 8:1.

In yet another aspect, the present disclosure further provides use of the lipid nanoparticle in preparing an mRNA vaccine.

In one embodiment, the lipid nanoparticle of the present disclosure may be prepared by using conventional methods in the art, such as microfluidic techniques.

In yet another aspect, the present disclosure further provides a medicament comprising the aforementioned bioactive substance and lipid nanoparticle.

Furthermore, the medicament further comprises a pharmaceutically acceptable excipient.

Furthermore, the pharmaceutically acceptable excipient of the present disclosure includes, for example, carriers, adjuvants, diluents, etc.

Furthermore, the medicament of the present disclosure is a gene drug.

Furthermore, the medicament of the present disclosure is a liquid formulation or a lyophilized powder formulation.

Furthermore, the medicament of the present disclosure is a formulation for oral administration, intramuscular injection, subcutaneous injection, intravenous injection, aerosol inhalation, or dry powder inhalation.

In yet another aspect, the present disclosure further provides a method for delivering a bioactive substance, which comprises administering to an individual in need thereof the medicament of the present disclosure.

The present disclosure has the following beneficial effects:
The present disclosure provides a steroid-cationic lipid compound having a structure represented by formula (I) and use thereof. The compound can be mixed with a helper lipid and a polyethylene glycol lipid to prepare a lipid nanoparticle for delivering a bioactive substance. The three-component LNP prepared by using the steroid-cationic lipid compound of the present disclosure has the advantages of a simple preparation process, good stability, and high transfection efficiency. The three-component LNP is used for delivering a nucleic acid, such as an mRNA, which can efficiently and stably deliver the nucleic acid mRNA to a target cell or organ, leading to a high specific antibody response in an animal, and having better safety.

The three-component LNP prepared by using the steroid-cationic lipid compound provided in the present disclosure has better stability in the lyophilization process and is more suitable for the development and application of an mRNA-LNP lyophilized powder formulation. In addition, the three-component LNP prepared by using the steroid-cationic lipid compound provided in the present disclosure can still retain the structural stability and efficient transfection activity after nebulization, thereby having good prospects for use in the aerosol inhalation administration or dry powder inhalation administration of mRNA.

### BRIEF DESCRIPTION OF THE DRAWINGS

FIG. 1 shows the expression of GFP-mRNA-LNPs in Hep3B cells as detected by fluorescence microscopy;
FIG. 2 shows the serum antibody titers, as detected by ELISA, after mice were immunized with mRNA-LNPs;
FIG. 3a shows the CD8T cell immunity, as detected by ICS, after mice were immunized with mRNA-LNPs;
FIG. 3b shows the CD4T cell immunity, as detected by ICS, after mice were immunized with mRNA-LNPs;
FIG. 4 shows the serum IL-6 expression in mice after high-dose administration of mRNA-LNPs;
FIG. 5 shows the comparison of DLS before and after LNP nebulization;
FIG. 6 shows the expression of GFP fluorescent protein after transfection of GFP-mRNA-LNPs into Hep3B cells before and after nebulization as detected by fluorescence microscopy;
FIG. 7a shows the *in vivo* and organ images of mice after aerosol inhalation of Luc-mRNA-LNPs;
FIG. 7b shows the *in vivo* and organ imaging of mice after aerosol inhalation of Luc-mRNA-LNPs.

### DETAILED DESCRIPTION

The technical solutions in the examples of the present disclosure will be described clearly and completely below with reference to the drawings in the examples of the present disclosure. It is apparent that the described examples are only a part of the examples of the present disclosure, rather than all of them. Based on the examples of the present disclosure, all other examples obtained by those of ordinary skill in the art without creative work shall fall within the protection scope of the present disclosure.

### Definitions

As used in this specification, the following words and phrases are generally intended to have the meanings as set forth below, unless the context in which they are used indicates otherwise.

As used herein, the term "lipid nanoparticle" or "LNP" refers to a particle with a nanoscale size (e.g., 1 nm to 1,000 nm) comprising one or more types of lipid molecules.

As used herein, the term "gene drug" generally consists of a carrier or a delivery system comprising an engineered gene construct. Its active ingredient may be a DNA, an RNA, or a genetically modified virus, bacterium, or cell. By introducing an exogenous gene into a target cell or tissue, a specific gene is replaced, compensated, blocked, or corrected, thereby achieving the purposes of treating and preventing diseases.

As used herein, the term "nucleic acid" refers to a polymer containing at least two kinds of deoxyribonucleotides or ribonucleotides in either single- or double-stranded form and includes a DNA, an RNA, and a hybrid thereof.

As used herein, the term "lipid compound" or "lipid" refers to a group of organic compounds that include, but are not limited to, esters of fatty acids and are generally characterized by being poorly soluble in water, but soluble in many organic solvents. The organic solvents of the present disclosure include, but are not limited to: benzene, toluene, pentane, hexane, methanol, ethanol, isopropanol, diethyl ether, ethyl acetate, acetone, and tetrachloromethane.

As used herein, the term "alkyl" refers to a saturated linear or branched hydrocarbon group. As used herein, C₁₋₉ alkyl includes linear or branched alkyl containing 1, 2, 3, 4, 5, 6, 7, 8, or 9 carbon atoms, for example, including but not limited to, C₁₋₈, C₂₋₇, C₂₋₈, C₃₋₆, or C₄₋₇ alkyl; C₆₋₂₄ alkyl includes linear or branched alkyl containing 6, 7, 8, 9, 10, 11, 12, 13, 14, 15, 16, 17, 18, 19, 20, 21, 22, 23, or 24 carbon atoms, for example, including but not limited to, C₆₋₈, C₆₋₁₇, C₆₋₂₂, C₁₀₋₁₆, or C₁₄₋₁₇ alkyl; C₁₋₅ alkyl includes linear or branched alkyl containing 1, 2, 3, 4, or 5 carbon atoms, for example, including but not limited to, C₁₋₄, C₂₋₄, C₂₋₃, C₃₋₅, or C₄₋₅ alkyl; C₁₋₂₀ alkyl includes linear or branched alkyl containing 1, 2, 3, 4, 5, 6, 7, 8, 9, 10, 11, 12, 13, 14, 15, 16, 17, 18, 19, or 20 carbon atoms, for example, including but not limited to, C₁₋₈, C₁₋₁₀, C₆₋₂₂, C₁₀₋₁₆, or C₁₄₋₁₇ alkyl.

As used herein, the term "alkenyl" refers to an unsaturated linear or branched hydrocarbon group containing one or more unsaturated carbon-carbon double bonds. The unsaturated carbon-carbon double bond may be present at any stable point along the chain. As used herein, C₂₋₉ alkenyl includes linear or branched alkenyl containing 2, 3, 4, 5, 6, 7, 8, or 9 carbon atoms, for example, including but not limited to, C₁₋₈, C₂₋₇, C₂₋₈, C₃₋₆, or C₄₋₈ linear or branched alkenyl; C₆₋₂₄ alkenyl includes linear or branched alkenyl containing 6, 7, 8, 9, 10, 11, 12, 13, 14, 15, 16, 17, 18, 19, 20, 21, 22, 23, or 24 carbon atoms, for example, including but not limited to, C₆₋₈, C₆₋₁₇, C₆₋₂₂, C₁₀₋₁₆, or C₁₄₋₁₇ alkenyl. As used herein, the term "alkynyl" refers to an unsaturated linear or branched hydrocarbon group containing one or more unsaturated carbon-carbon triple bonds. As used herein, C₂₋₉ alkynyl includes linear or branched alkynyl containing 2, 3, 4, 5, 6, 7, 8, or 9 carbon atoms, for example, including but not limited to, C₁₋₈, C₂₋₇, C₂₋₈, C₃₋₆, or C₄₋₇ alkynyl.

As used herein, one or more carbons at positions other than terminal positions of alkyl, alkenyl, and alkynyl may be substituted with heteroatoms such as nitrogen, oxygen, sulfur, and oxides thereof such as nitrogen oxides, carbonyl, a sulfone (sulfoxide) group; for example,-(CH₂)ₘ-O-(CH₂)ₙ-, -(CH₂)ₘ-S-(CH₂)ₙ-, -(CH₂)ₘ-S-S-(CH₂)ₙ-, -(CH₂)ₘ-CO-(CH₂)ₙ-, -(CH₂)ₘ-OCO-(CH₂)ₙ-, or -(CH₂)ₘ-OCOO-(CH₂)ₙ- (wherein m and n may be integers from 1 to 9) may be unsubstituted or substituted with one or more heteroatom substituents as described herein. In certain embodiments, the alkyl, alkenyl, or alkynyl does not contain any heteroatoms therein.

As used herein, the term "alkylene" refers to a saturated divalent linear or branched hydrocarbon group. As used herein, C₁₋₉ alkylene includes linear or branched alkylene containing 1, 2, 3, 4, 5, 6, 7, 8, or 9 carbon atoms, for example, including but not limited to, C₁₋₈, C₂₋₇, C₂₋₈, C₃₋₆, or C₄₋₇ alkylene; C₁₋₁₂ alkylene includes linear or branched alkylene containing 1, 2, 3, 4, 5, 6, 7, 8, 9, 10, 11, or 12 carbon atoms, for example, including but not limited to, C₁₋₈, C₂₋₇, C₂₋₈, C₃₋₆, or C₄₋₁₂ alkylene; C₁₋₁₀ alkylene includes linear or branched alkylene containing 1, 2, 3, 4, 5, 6, 7, 8, 9, or 10 carbon atoms, for example, including but not limited to, C₁₋₈, C₂₋₇, C₂₋₈, C₃₋₆, or C₄₋₁₀ alkylene; C₁₋₆ alkylene includes linear or branched alkylene containing 1, 2, 3, 4, 5, or 6 carbon atoms, for example, including but not limited to, C₁₋₅, C₂₋₄, C₃₋₆, or C₄₋₆ alkylene.

As used herein, the term "alkenylene" refers to an unsaturated linear or branched hydrocarbon group containing one or more unsaturated carbon-carbon double bonds. As used herein, C₂₋₉ alkenylene includes linear or branched alkenylene containing 2, 3, 4, 5, 6, 7, 8, or 9 carbon atoms, for example, including but not limited to, C₂₋₇, C₂₋₈, C₃₋₆, or C₄₋₇ alkenylene; C₂₋₁₂ alkenylene includes linear or branched alkenylene containing 2, 3, 4, 5, 6, 7, 8, 9, 10, 11, or 12 carbon atoms, for example, including but not limited to, C₂₋₈, C₂₋₇, C₃₋₆, or C₄₋₁₂ alkenylene; C₂₋₆ alkenylene includes linear or branched alkenylene containing 2, 3, 4, 5, or 6 carbon atoms, for example, including but not limited to, C₂₋₅, C₂₋₄, C₃₋₆, or C₄₋₆ alkenylene.

As used herein, the term "alkynylene" refers to an unsaturated linear or branched hydrocarbon group containing one or more unsaturated carbon-carbon triple bonds. As used herein, C₂₋₁₂ alkynylene includes linear or branched alkynylene containing 2, 3, 4, 5, 6, 7, 8, 9, 10, 11, or 12 carbon atoms, for example, including but not limited to, C₂₋₈, C₂₋₇, C₃₋₆, or C₄₋₁₂ alkynylene; C₃₋₉ alkynylene includes linear or branched alkynylene containing 3, 4, 5, 6, 7, 8, or 9 carbon atoms, for example, including but not limited to, C₃₋₆, C₃₋₈, or C₄₋₇ alkynylene.

As used herein, the alkylene, alkenylene, or alkynylene may contain one or more cyclic aliphatic groups and/or one or more heteroatoms such as oxygen, nitrogen, or sulfur, and may be optionally substituted with one or more substituents such as alkyl, halogen, alkoxy, hydroxy, amino, aryl, ether, ester, or amide. One or more carbons at positions other than terminal positions may be substituted with heteroatoms such as nitrogen, oxygen, sulfur, and oxides thereof such as nitrogen oxides, carbonyl, a sulfone or sulfoxide group. In certain embodiments, the alkylene, alkenylene, or alkynylene is unsubstituted. In certain embodiments, the alkylene, alkenylene, or alkynylene does not contain any heteroatoms.

As used herein, the term "nitrogen-containing heterocyclic group" is a heterocyclic group containing a nitrogen atom in the structure, including but not limited to, substituted or unsubstituted aziridinyl, azetidinyl, β-propiolactamyl, pyrrolyl, piperidinylalkyl, pyrazolyl, imidazolyl, oxazolyl, isoxazolyl, thiazolyl, pyridinyl, caprolactamyl, pyranyl, pyridazinyl, pyrimidinyl, pyrazinyl, piperazinyl, piperazinylalkyl, indolyl, benzimidazolyl, carbazolyl, quinolinyl, isoquinolinyl, pteridinyl, acridinyl, 7H-purinyl, phenazinyl, phenothiazinyl, or 1H-azepinyl.

As used herein, the term "alkoxy" refers to an "alkyl-O-" group, wherein the alkyl is as defined herein. As used herein, C₆₋₂₄ alkoxy includes linear or branched alkoxy containing 6, 7, 8, 9, 10, 11, 12, 13, 14, 15, 16, 17, 18, 19, 20, 21, 22, 23, or 24 carbon atoms, for example, including but not limited to, C₆₋₈, C₆₋₁₇, C₆₋₂₂, C₁₀₋₁₆, or C₁₄₋₁₇ alkoxy.

As used herein, the term "cycloalkyl" refers to a saturated cyclic hydrocarbon group. As used herein, C₃₋₈ cycloalkyl includes cycloalkyl containing 3, 4, 5, 6, 7, or 8 carbon atoms, for example, including but not limited to, C₃₋₇, C₄₋₇, or C₃₋₆ cycloalkyl.

As used herein, the term "cycloalkenyl" refers to a cyclic hydrocarbon group containing at least one carbon-carbon double bond. As used herein, C₃₋₈ cycloalkenyl includes cycloalkenyl containing 3, 4, 5, 6, 7, or 8 carbon atoms, for example, including but not limited to, C₃₋₇, C₄₋₇, or C₃₋₆ cycloalkenyl. As used herein, the term "cycloalkynyl" refers to a cyclic hydrocarbon group containing at least one carbon-carbon triple bond. As used herein, C₃₋₈ cycloalkynyl includes cycloalkynyl containing 3, 4, 5, 6, 7, or 8 carbon atoms, for example, including but not limited to, C₃₋₇, C₄₋₇, or C₃₋₆ cycloalkynyl. As used herein, the term "cycloalkylene" refers to divalent cycloalkyl that is used to link two structures together. As used herein, C₃₋₁₂ cycloalkylene includes cycloalkylene containing 3, 4, 5, 6, 7, 8, 9, 10, 11, or 12 carbon atoms, for example, including but not limited to, C₃₋₈, C₄₋₇, C₅₋₈, or C₆₋₁₂ cycloalkylene; C₃₋₈ cycloalkylene includes cycloalkyl containing 3, 4, 5, 6, 7, or 8 carbon atoms, for example, including but not limited to, C₃₋₇, C₄₋₇, or C₆₋₈ cycloalkylene.

As used herein, the term "cycloalkenylene" refers to divalent cycloalkenyl that is used to link two structures together. As used herein, C₃₋₁₂ cycloalkenylene includes cycloalkenylene containing 3, 4, 5, 6, 7, 8, 9, 10, 11, or 12 carbon atoms, for example, including but not limited to, C₃₋₈, C₄₋₇, C₅₋₈, or C₆₋₁₂ cycloalkenylene; C₃₋₈ cycloalkenylene includes cycloalkenylene containing 3, 4, 5, 6, 7, or 8 carbon atoms, for example, including but not limited to, C₃₋₇, C₄₋₇, or C₆₋₈ cycloalkenylene.

As used herein, the term "cycloalkynylene" refers to divalent cycloalkynyl that is used to link two structures together. As used herein, C₃₋₁₂ cycloalkynylene includes cycloalkynylene containing 3, 4, 5, 6, 7, 8, 9, 10, 11, or 12 carbon atoms, for example, including but not limited to, C₃₋₈, C₄₋₇, C₅₋₈, or C₆₋₁₂ cycloalkynylene; C₃₋₈ cycloalkynylene includes cycloalkynylene containing 3, 4, 5, 6, 7, or 8 carbon atoms, for example, including but not limited to, C₃₋₇, C₄₋₇, or C₆₋₈ cycloalkynylene.

As used herein, the term "arylene" refers to divalent aryl that is used to link two structures together, wherein the aryl includes monocyclic aromatic (having, for example, 4n+2 delocalized electrons) groups and polycyclic aromatic groups. As used herein, C₆₋₁₂ arylene includes arylene containing 6, 7, 8, 9, 10, 11, or 12 carbon atoms, for example, including but not limited to, C₆₋₈, C₆₋₉, or C₆₋₁₂ arylene. As used herein, the term "cationic lipid" refers to a lipid molecule that is positively charged in response to ambient pH or hydrogen ion activity.

As used herein, the term "helper lipid" refers to a lipid that is not positively charged at ambient pH, including neutral lipids that are not charged at all, zwitterionic lipids, and anionic lipids that are negatively charged.

As used herein, the term "polyethylene glycol lipid" refers to a lipid molecule comprising a lipid portion and a polyethylene glycol portion.

As used herein, the term "delivery system" refers to a formulation or composition that regulates the spatial, temporal, and dose distribution of a bioactive ingredient in an organism.

It should be noted that the methods used in the present disclosure are all conventional methods unless otherwise specified, and the reagents used in the present disclosure are all commercially available products unless otherwise specified.

### Examples

### Example 1. Synthesis of Compound 1

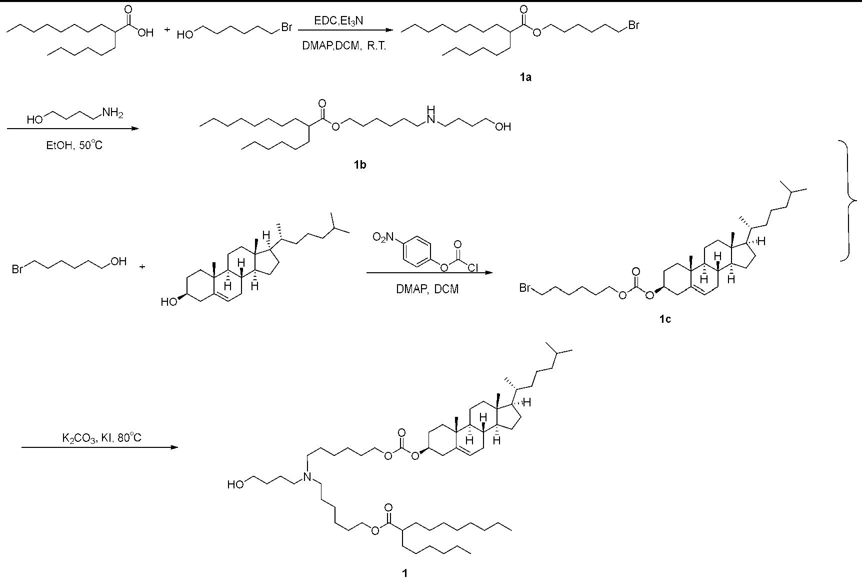

### Step 1: Synthesis of 6-bromohexyl 2-hexyldecanoate (1a)

2-Hexyldecanoic acid (2.12 g, 5.0 mmol) was dissolved in dichloromethane (30 mL), and 6-bromohexanol (0.93 g, 5.0 mmol), 4-dimethylaminopyridine (DMAP, 0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 1a (1.45 g, light yellow oil, yield: 70%).
MS m/z (ESI): 419.2 [M+1]

### Step 2: Synthesis of 6-((4-hydroxybutyl)amino)hexyl 2-hexyldecanoate (1b)

At room temperature, 6-bromohexyl 2-hexyldecanoate 1a (1.28 g, 3 mmol) was dissolved in ethanol (20 mL), and 4-amino-1-butanol (4.00 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 1b (1.13 g, light yellow oil).
MS m/z (ESI): 428.4 [M+1]

### Step 3: Synthesis of 6-bromohexyl cholesterol carbonate (1c)

6-Bromohexanol (0.91 g, 5.0 mmol) was dissolved in dichloromethane (30 mL), 4-dimethylaminopyridine (1.22 g, 10 mmol) was added, and then 4-nitrophenyl chloroformate (1.11 g, 5.5 mmol) was added in portions. The mixture was stirred and reacted at room temperature for 3 h. Cholesterol (2.16 g, 5.6 mmol) was added to the reaction solution, and the resulting mixture was stirred at room temperature overnight. After the reaction was completed as detected by TLC, dichloromethane (20 mL) was added for dilution, and then the resulting mixture was washed with saturated brine (30 mL). The organic phase was dried over anhydrous sodium sulfate, filtered, and concentrated to give product 1c (1.83 g, light yellow oil, yield: 62%).
MS m/z (ESI): 593.3 [M+1]

### Step 4: Synthesis of compound 1

6-((4-Hydroxybutyl)amino)hexyl 2-hexyldecanoate 1b (428 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 1 (470 mg, light yellow oil, yield: 50%).
MS m/z (ESI): 941.4 [M+1]
1H NMR (300MHz, CDCl₃): δ 5.40 (t, 1H, J = 5.4 Hz), 4.53-4.40 (m, 1H), 4.20-4.02 (m, 4H), 3.67-3.61 (m, 2H), 3.58-3.50 (m, 2H), 2.47-2.42 (m, 8H), 2.03-1.72 (m, 5H), 1.67-0.85 (m, 82H), 0.70 (s, 3H)

### Example 2. Synthesis of Compound 2

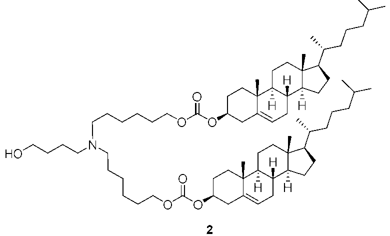

6-Bromohexyl cholesterol carbonate 1c (1.48 g, 2.5 mmol) was dissolved in tetrahydrofuran, and acetonitrile, 4-amino-1-butanol (89.2 mg, 1.0 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 2 (679 mg, light yellow solid, yield: 61%).
MS m/z (ESI): 1115.2 [M+1]
1H NMR (300MHz, CDCl₃): δ 5.40 (t, 2H, J = 5.4 Hz), 4.53-4.40 (m, 2H), 4.15 (t, 4H, J = 5.6 Hz), 3.76 (t, 2H, J = 5.4 Hz), 3.20-3.01 (m, 6H), 2.45-2.31 (m, 4H), 2.02-1.75 (m, 16H), 1.74-1.22 (m, 36H), 1.19-0.87 (m, 44H), 0.70 (s, 6H)

### Example 3. Synthesis of Compound 3

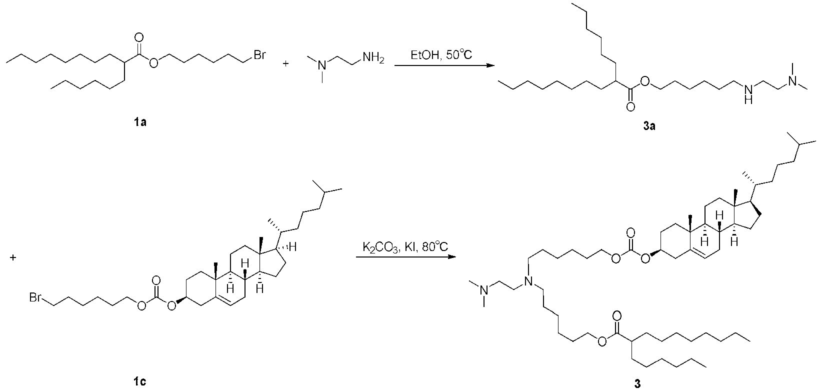

### Step 1: Synthesis of 6-((2-(dimethylamino)ethyl)amino)hexyl 2-hexyldecanoate (3a)

At room temperature, 6-bromohexyl 2-hexyldecanoate 1a (1.28 g, 3 mmol) was dissolved in ethanol (20 mL), and N,N-dimethylethylenediamine (4.00 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 3a (1.20 g, light yellow oil).
MS m/z (ESI): 427.4 [M+1]

### Step 2: Synthesis of compound 3

6-((2-(Dimethylamino)ethyl)amino)hexyl 2-hexyldecanoate 3a (426 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 3 (517 mg, light yellow oil, yield: 55%).
MS m/z (ESI): 940.0 [M+1]
1H NMR (300MHz, CDCl₃): δ 5.40 (t, 1H, J = 5.4 Hz), 4.53-4.40 (m, 1H), 4.20-4.02 (m, 3H), 3.76-3.61 (m, 2H), 3.45-3.38 (m, 3H), 2.94-2.85 (m, 1H), 2.55-2.27 (m, 5H), 2.03-0.85 (m, 70H), 0.70 (s, 3H)

### Example 4. Synthesis of Compound 4

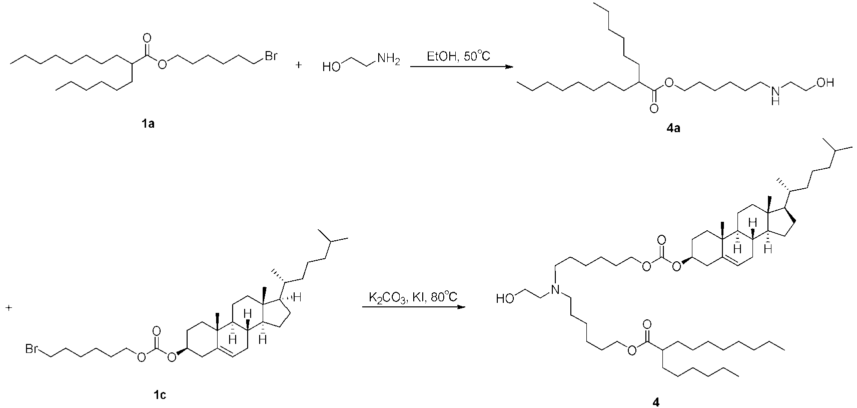

### Step 1: Synthesis of 6-((2-hydroxyethyl)amino)hexyl 2-hexyldecanoate (4a)

At room temperature, 6-bromohexyl 2-hexyldecanoate 1a (1.28 g, 3 mmol) was dissolved in ethanol (20 mL), and ethanolamine (2.75 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 4a (1.01 g, light yellow oil).
MS m/z (ESI): 400.4 [M+1]

### Step 2: Synthesis of compound 4

6-((2-Hydroxyethyl)amino)hexyl 2-hexyldecanoate 4a (400 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 4 (474 mg, light yellow oil, yield: 52%).
MS m/z (ESI): 913.1 [M+1]
1H NMR (300MHz, CDCl₃): δ 5.40 (t, 1H, J = 5.4 Hz), 4.53-4.40 (m, 1H), 4.20-4.02 (m, 4H), 3.67-3.61 (m, 2H), 3.58-3.50 (m, 4H), 2.47-2.42 (m, 2H), 2.03-1.72 (m, 5H), 1.67-0.85 (m, 82H), 0.70 (s, 3H)

### Example 5. Synthesis of Compound 5

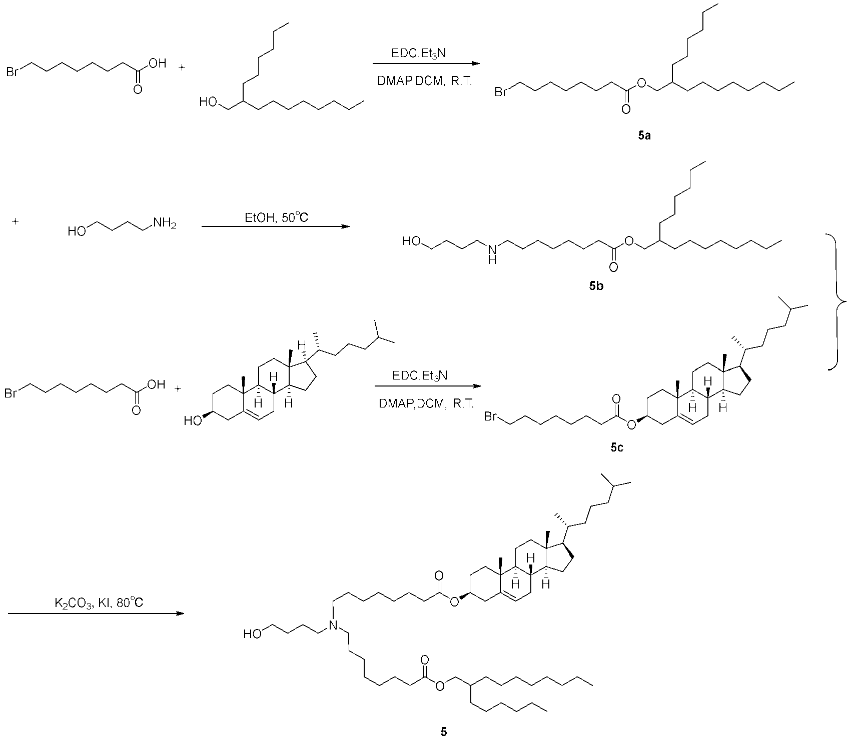

### Step 1: Synthesis of 2-hexyldecyl 8-bromooctanoate (5a)

8-Bromooctanoic acid (1.12 g, 5.0 mmol) was dissolved in dichloromethane (30 mL), and 2-hexyldecanol (1.21 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 5a (1.52 g, light yellow oil, yield: 68%).
MS m/z (ESI): 447.2 [M+1]

### Step 2: Synthesis of 2-hexyldecyl 8-((4-hydroxybutyl)amino)octanoate (5b)

At room temperature, 2-hexyldecyl 8-bromooctanoate 5a (1.34 g, 3 mmol) was dissolved in ethanol (20 mL), and butanolamine (4.00 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 5b (1.21 g, light yellow oil).
MS m/z (ESI): 456.4 [M+1]

### Step 3: Synthesis of cholesterol 8-bromooctanoate (5c)

8-Bromooctanoic acid (1.12 g, 5.0 mmol) was dissolved in dichloromethane (30 mL), and cholesterol (1.93 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 5c (1.77 g, light yellow oil, yield: 60%).
MS m/z (ESI): 591.4 [M+1]

### Step 4: Synthesis of compound 5

2-Hexyldecyl 8-((4-hydroxybutyl)amino)octanoate 5b (455 mg, 1.0 mmol) was dissolved in tetrahydrofuran, and acetonitrile, cholesterol 8-bromooctanoate 5c (708 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 5 (464 mg, light yellow oil, yield: 48%).
MS m/z (ESI): 967.6 [M+1]
1H NMR (300MHz, CDCl₃): δ 5.40 (t, 1H, J = 5.4 Hz), 4.53-4.40 (m, 1H), 4.20-4.02 (m, 2H), 3.67-3.61 (m, 2H), 2.64 (t, 6H, J = 5.4 Hz), 2.37-2.25 (m, 6H), 2.10-0.85 (m, 93H), 0.70 (s, 3H)

### Example 6. Synthesis of Compound 6

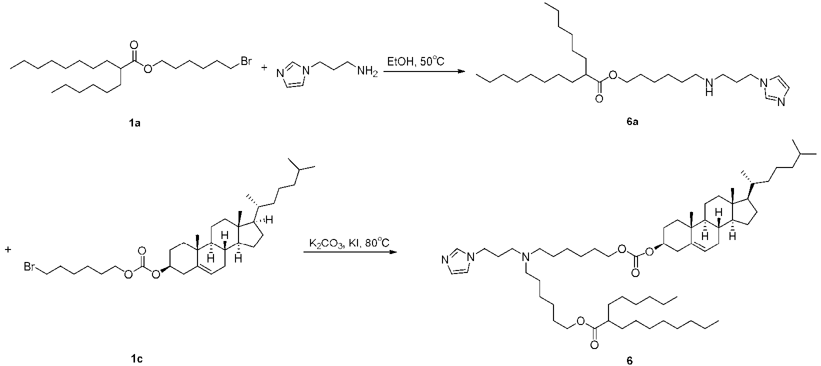

### Step 1: Synthesis of 6-((3-(1H-imidazol-1-yl)propyl)amino)hexyl 2-hexyldecanoate (6a)

At room temperature, 6-bromohexyl 2-hexyldecanoate 1a (1.28 g, 3 mmol) was dissolved in ethanol (20 mL), and 1-(3-aminopropyl)imidazole (5.63 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 6a (1.46 g, light yellow oil).
MS m/z (ESI): 464.4 [M+1]

### Step 2: Synthesis of compound 6

6-((3-(1H-Imidazol-1-yl)propyl)amino)hexyl 2-hexyldecanoate 6a (463 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 6 (517 mg, light yellow oil, yield: 53%).
MS m/z (ESI): 976.9 [M+1]
1H NMR (300MHz, CDCl₃): δ 7.49 (s, 1H), 7.08 (d, 1H, J = 5.6 Hz), 6.93 (d, 1H, J = 5.6 Hz), 5.40 (t, 1H, J = 5.4 Hz), 4.53-4.40 (m, 1H), 4.20-3.95 (m, 5H), 2.47-2.30 (m, 9H), 2.36-0.85 (m, 87H), 0.70 (s, 3H)

### Example 7. Synthesis of Compound 7

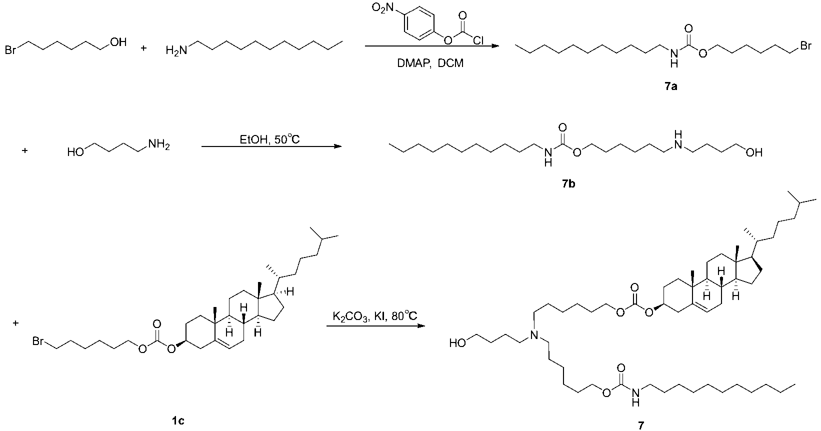

### Step 1: Synthesis of 6-bromohexyl undecylcarbamate (7a)

6-Bromohexanol (0.91 g, 5.0 mmol) was dissolved in dichloromethane (30 mL), 4-dimethylaminopyridine (1.22 g, 10 mmol) was added, and then 4-nitrophenyl chloroformate (1.11 g, 5.5 mmol) was added in portions. The mixture was stirred and reacted at room temperature for 3 h. Undecylamine (0.96 g, 5.6 mmol) was added to the reaction solution, and the resulting mixture was stirred at room temperature overnight. After the reaction was completed as detected by TLC, dichloromethane (20 mL) was added for dilution, and then the resulting mixture was washed with saturated brine (30 mL). The organic phase was dried over anhydrous sodium sulfate, filtered, and concentrated to give compound 7a (1.23 g, light yellow oil, yield: 65%).
MS m/z (ESI): 378.2 [M+1]

### Step 2: Synthesis of 6-((4-hydroxybutyl)amino)hexyl undecylcarbamate (7b)

At room temperature, 6-bromohexyl undecylcarbamate 7a (1.13 g, 3 mmol) was dissolved in ethanol (20 mL), and butanolamine (4.00 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 7b (1.14 g, light yellow oil).
MS m/z (ESI): 387.4 [M+1]

### Step 3: Synthesis of compound 7

6-((4-Hydroxybutyl)amino)hexyl undecylcarbamate 7b (386 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 7 (459 mg, light yellow oil, yield: 51%).
MS m/z (ESI): 900.4 [M+1]
1H NMR (300MHz, CDCl₃): δ 5.42 (t, 1H, J = 5.4 Hz), 4.80-4.71 (m, 1H), 4.51-4.40 (m, 1H), 4.20-4.02 (m, 4H), 3.75-3.61 (m, 2H), 3.20-3.11 (m, 2H), 2.91-2.73 (m, 5H), 2.43-2.36 (m, 2H), 2.10-0.83 (m, 79H), 0.70 (s, 3H)

### Example 8. Synthesis of Compound 8

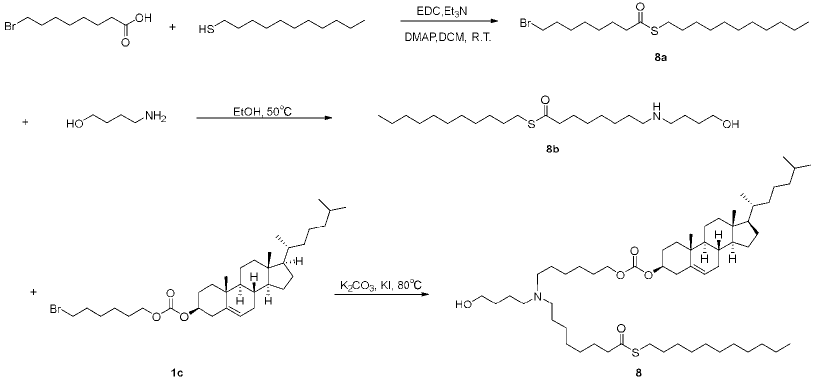

### Step 1: Synthesis of S-undecyl 8-bromooctanethioate (8a)

8-Bromooctanoic acid (1.12 g, 5.0 mmol) was dissolved in dichloromethane (30 mL), and undecanethiol (0.94 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 8a (1.30 g, light yellow oil, yield: 66%).
MS m/z (ESI): 393.2 [M+1]

### Step 2: Synthesis of S-undecyl 8-((4-hydroxybutyl)amino)octanethioate (8b)

At room temperature, S-undecyl 8-bromooctanethioate 8a (1.18 g, 3 mmol) was dissolved in ethanol (20 mL), and butanolamine (4.00 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 8b (1.18 g, light yellow oil).
MS m/z (ESI): 402.3 [M+1]

### Step 3: Synthesis of compound 8

S-Undecyl 8-((4-hydroxybutyl)amino)octanethioate 8b (401 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 8 (458 mg, light yellow oil, yield: 50%).
MS m/z (ESI): 914.9 [M+1]
1H NMR (300MHz, CDCl₃): δ 5.42 (t, 1H, J = 5.4 Hz), 4.51-4.40 (m, 1H), 4.20-4.02 (m, 2H), 3.65-3.51 (m, 2H), 2.91-2.83 (m, 2H), 2.60-2.36 (m, 10H), 2.10-0.86 (m, 82H), 0.70 (s, 3H)

### Example 9. Synthesis of Compound 9

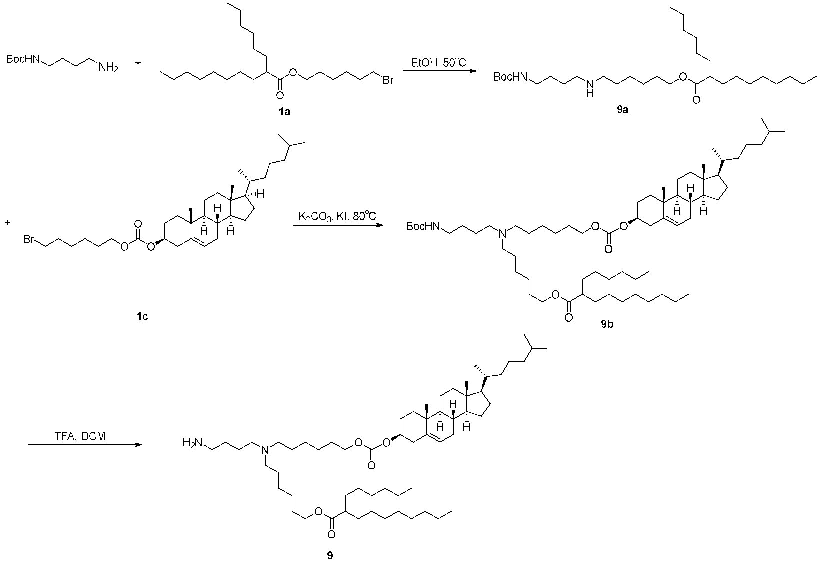

### Step 1: Synthesis of 6-((4-((tert-butoxycarbonyl)amino)butyl)amino)hexyl 2-hexyldecanoate (9a)

At room temperature, 6-bromohexyl 2-hexyldecanoate 1a (1.28 g, 3 mmol) was dissolved in ethanol (20 mL), and BOC-1,4-butanediamine hydrochloride (10.1 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 9a (1.51 g, light yellow oil).
MS m/z (ESI): 527.5 [M+1]

### Step 2: Synthesis of compound 9b

6-((4-((tert-Butoxycarbonyl)amino)butyl)amino)hexyl 2-hexyldecanoate 9a (526 mg, 1.0 mmol) was dissolved in tetrahydrofuran, and acetonitrile, 6-bromohexyl cholesterol carbonate 1c (711 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 9b (540 mg, light yellow oil, yield: 52%).
MS m/z (ESI): 1039.9 [M+1]

### Step 3: Synthesis of compound 9

Compound 9b (1.04 g, 1.0 mmol) was dissolved in dichloromethane, and a solution of trifluoroacetic acid in dichloromethane was added in an ice bath. The mixture was stirred at room temperature for 16 h. Trifluoroacetic acid was removed by rotary evaporation, and dichloromethane was added for dissolution. The resulting mixture was washed twice with a saturated sodium bicarbonate solution. The organic phase was dried over anhydrous sodium sulfate, filtered, and concentrated, and the residue was separated by column chromatography to give compound 9 (460 mg, light yellow oil, yield: 49%).
MS m/z (ESI): 940.1 [M+1]
1H NMR (300MHz, CDCl₃): δ 5.40 (t, 1H, J = 5.4 Hz), 4.63-4.40 (m, 1H), 4.19-4.03 (m, 4H), 2.79-2.70 (m, 2H), 2.55-2.28 (m, 10H), 2.02-0.87 (m, 89H), 0.70 (s, 3H)

### Example 10. Synthesis of Compound 10

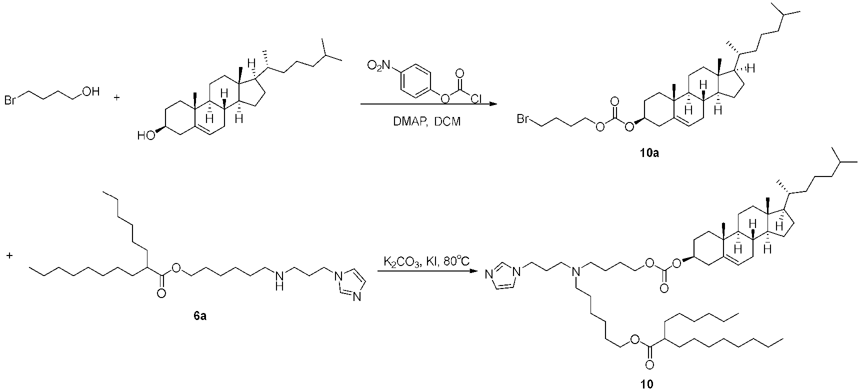

### Step 1: Synthesis of 4-bromobutyl cholesterol carbonate (10a)

4-Bromobutanol (0.77 g, 5.0 mmol) was dissolved in dichloromethane (30 mL), 4-dimethylaminopyridine (1.22 g, 10 mmol) was added, and then 4-nitrophenyl chloroformate (1.11 g, 5.5 mmol) was added in portions. The mixture was stirred and reacted at room temperature for 3 h. Cholesterol (2.16 g, 5.6 mmol) was added to the reaction solution, and the resulting mixture was stirred at room temperature overnight. After the reaction was completed as detected by TLC, dichloromethane (20 mL) was added for dilution, and then the resulting mixture was washed with saturated brine (30 mL). The organic phase was dried over anhydrous sodium sulfate, filtered, and concentrated to give product 10a (1.50 g, light yellow oil, yield: 53%).
MS m/z (ESI): 565.3 [M+1]

### Step 2: Synthesis of compound 10

6-((3-(1H-Imidazol-1-yl)propyl)amino)hexyl 2-hexyldecanoate 6a (463 mg, 1.0 mmol) was dissolved in tetrahydrofuran, and acetonitrile, 4-bromobutyl cholesterol carbonate 10a (678 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 10 (455 mg, light yellow oil, yield: 48%).
MS m/z (ESI): 948.9 [M+1]
1H NMR (300MHz, CDCl₃): δ 7.49 (s, 1H), 7.08 (d, 1H, J = 5.6 Hz), 6.93 (d, 1H, J = 5.6 Hz), 5.40 (t, 1H, J = 5.4 Hz), 4.53-4.40 (m, 1H), 4.20-3.95 (m, 6H), 2.47-2.30 (m, 8H), 2.36-0.85 (m, 83H), 0.70 (s, 3H)

### Example 11. Synthesis of Compound 11

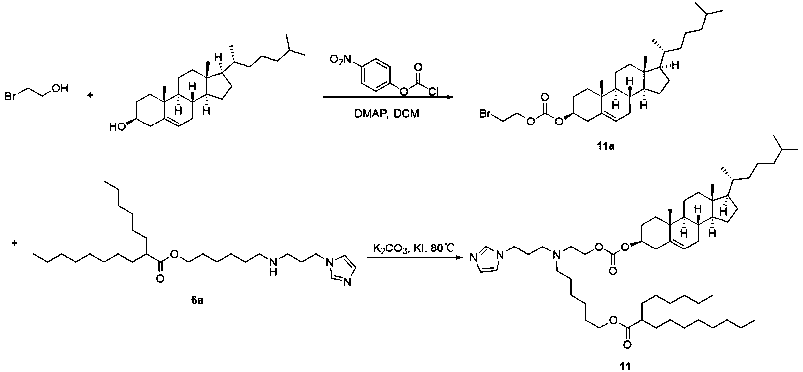

### Step 1: Synthesis of 2-bromoethyl cholesterol carbonate (11a)

2-Bromoethanol (0.63 g, 5.0 mmol) was dissolved in dichloromethane (30 mL), 4-dimethylaminopyridine (1.22 g, 10 mmol) was added, and then 4-nitrophenyl chloroformate (1.11 g, 5.5 mmol) was added in portions. The mixture was stirred and reacted at room temperature for 3 h. Cholesterol (2.16 g, 5.6 mmol) was added to the reaction solution, and the resulting mixture was stirred at room temperature overnight. After the reaction was completed as detected by TLC, dichloromethane (20 mL) was added for dilution, and then the resulting mixture was washed with saturated brine (30 mL). The organic phase was dried over anhydrous sodium sulfate, filtered, and concentrated to give product 11a (1.53 g, light yellow oil, yield: 57%).
MS m/z (ESI): 537.3 [M+1]

### Step 2: Synthesis of compound 11

6-((3-(1H-Imidazol-1-yl)propyl)amino)hexyl 2-hexyldecanoate 6a (463 mg, 1.0 mmol) was dissolved in tetrahydrofuran, and acetonitrile, 2-bromoethyl cholesterol carbonate 11a (644 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 11 (506 mg, light yellow oil, yield: 55%).
MS m/z (ESI): 920.8 [M+1]
1H NMR (300MHz, CDCl₃): δ 7.49 (s, 1H), 7.08 (d, 1H, J = 5.6 Hz), 6.93 (d, 1H, J = 5.6 Hz), 5.40 (t, 1H, J = 5.4 Hz), 4.53-4.40 (m, 1H), 4.20-3.95 (m, 6H), 2.74-2.365 (m, 2H), 2.47-2.30 (m, 6H), 2.36-0.85 (m, 79H), 0.70 (s, 3H)

### Example 12. Synthesis of Compound 12

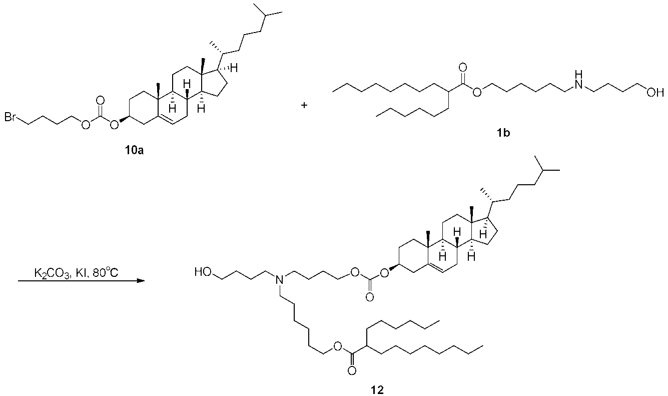

6-((4-Hydroxybutyl)amino)hexyl 2-hexyldecanoate 1b (428 mg, 1.0 mmol) was dissolved in tetrahydrofuran, and acetonitrile, 4-bromobutyl cholesterol carbonate 10a (679 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give product 12 (566 mg, light yellow oil, yield: 62%).
MS m/z (ESI): 913.0 [M+1]
1H NMR (300MHz, CDCl₃): δ 5.40 (t, 1H, J = 5.4 Hz), 4.53-4.40 (m, 1H), 4.20-4.02 (m, 4H), 3.67-3.61 (m, 2H), 2.63-2.32 (m, 8H), 2.03-1.72 (m, 5H), 1.67-0.85 (m, 80H), 0.70 (s, 3H)

### Example 13. Synthesis of Compound 13

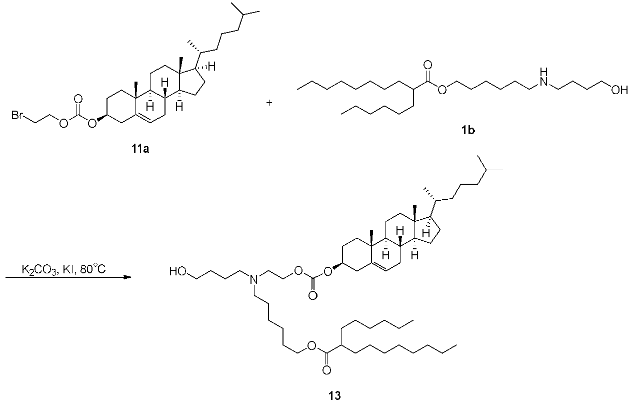

6-((4-Hydroxybutyl)amino)hexyl 2-hexyldecanoate 1b (428 mg, 1.0 mmol) was dissolved in tetrahydrofuran, and acetonitrile, 4-bromobutyl cholesterol carbonate 11a (645 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give product 13 (451 mg, light yellow oil, yield: 51%).
MS m/z (ESI): 885.1 [M+1]
1H NMR (300MHz, CDCl₃): δ 5.40 (t, 1H, J = 5.4 Hz), 4.53-4.40 (m, 1H), 4.29-4.21 (m, 2H), 4.20-4.02 (m, 2H), 3.67-3.61 (m, 2H), 2.85-2.78 (m, 2H), 2.63-2.32 (m, 6H), 2.03-1.72 (m, 5H), 1.67-0.85 (m, 76H), 0.70 (s, 3H)

### Example 14. Synthesis of Compound 14

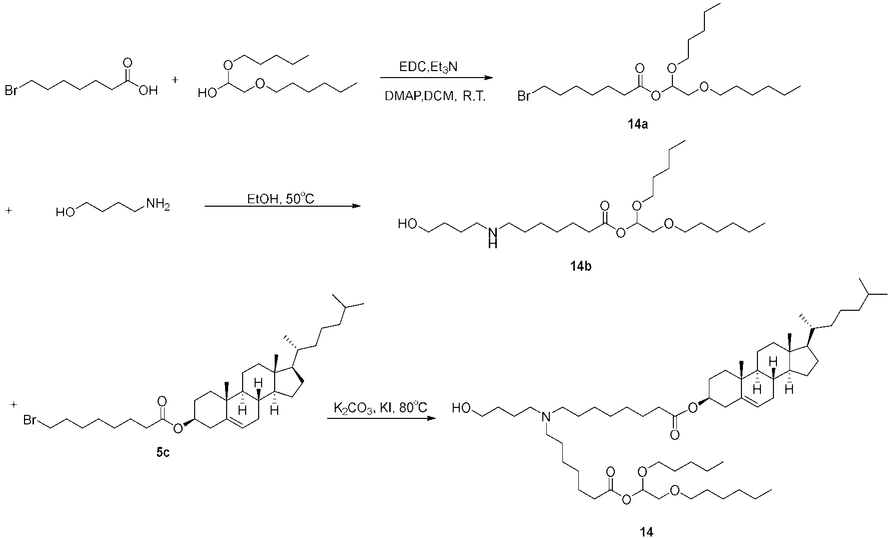

### Step 1: Synthesis of 2-(hexyloxy)-1-(pentyloxy)ethyl 7-bromoheptanoate (14a)

7-Bromoheptanoic acid (1.05 g, 5.0 mmol) was dissolved in dichloromethane (30 mL), and 2-(hexyloxy)-1-(pentyloxy)-1-ethanol (1.16 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 14a (1.08 g, light yellow oil, yield: 51%).
MS m/z (ESI): 423.2 [M+1]

### Step 2: Synthesis of 2-(hexyloxy)-1-(pentyloxy)ethyl 7-((4-hydroxybutyl)amino)heptanoate (14b)

At room temperature, 2-(hexyloxy)-1-(pentyloxy)ethyl 7-bromoheptanoate 14a (1.27 g, 3 mmol) was dissolved in ethanol (20 mL), and butanolamine (4.00 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 14b (1.05 g, light yellow oil).
MS m/z (ESI): 432.4 [M+1]

### Step 3: Synthesis of compound 14

2-(Hexyloxy)-1-(pentyloxy)ethyl 7-((4-hydroxybutyl)amino)heptanoate 14b (432 mg, 1.0 mmol) was dissolved in tetrahydrofuran, and acetonitrile, cholesterol 8-bromooctanoate 5c (708 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 14 (490 mg, light yellow oil, yield: 52%).
MS m/z (ESI): 942.8 [M+1]
1H NMR (300MHz, CDCl₃): δ 6.67 (t, 1H, J= 6.3 Hz), 5.40 (t, 1H, J = 5.4 Hz), 4.53-4.40 (m, 1H), 4.20-4.02 (m, 2H), 3.67-3.61 (m, 2H), 3.47-3.31 (m, 4H), 2.64 (t, 6H, J = 5.4 Hz), 2.37-2.25 (m, 6H), 2.10-0.85 (m, 80H), 0.70 (s, 3H)

### Example 15. Synthesis of Compound 15

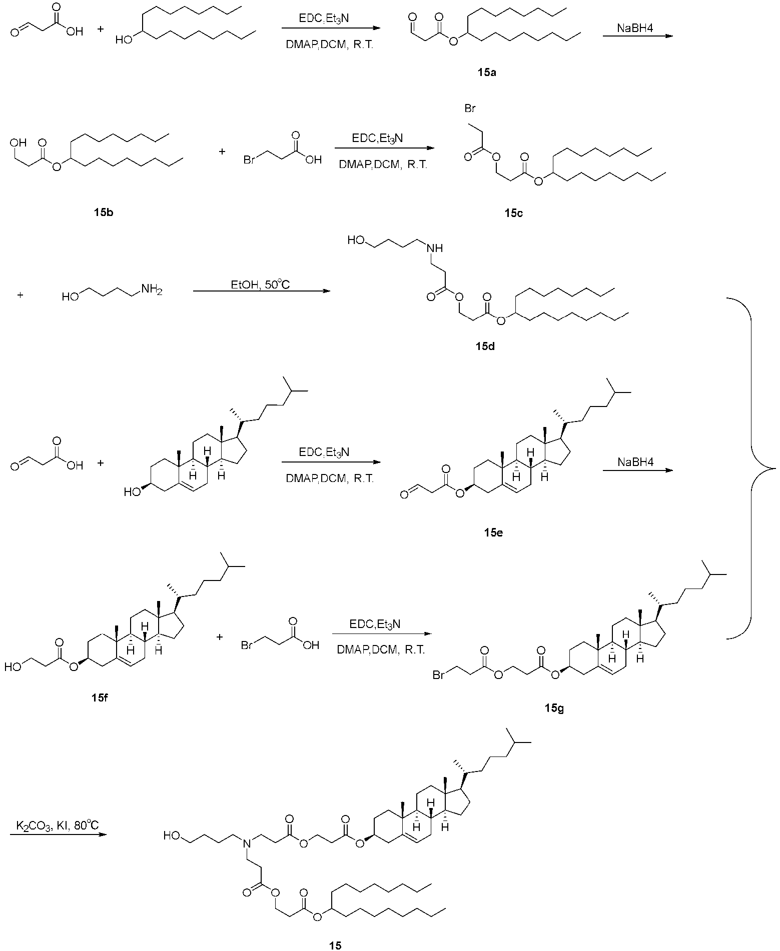

### Step 1: Synthesis of heptadecan-9-yl 3-oxopropanoate (15a)

3-Oxopropanoic acid (0.44 g, 5.0 mmol) was dissolved in dichloromethane (20 mL), and 9-heptadecanol (1.28 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 15a (0.78 g, light yellow oil, yield: 48%).
MS m/z (ESI): 327.3 [M+1]

### Step 2: Synthesis of heptadecan-9-yl 3-hydroxypropanoate (15b)

Heptadecan-9-yl 3-oxopropanoate 15a (0.78 g, 2.3 mmol) was dissolved in n-butanol (10 mL), and sodium borohydride (266 mg, 7 mmol) was added at 0 °C. The mixture was stirred at room temperature for 12 h. After the starting materials were consumed completely as monitored, a saturated ammonium chloride solution was added to quench the reaction, and the resulting mixture was extracted with dichloromethane. The organic phase was dried over anhydrous sodium sulfate and concentrated to give compound 15b (0.70 g, light yellow oil).
MS m/z (ESI): 329.3 [M+1]

### Step 3: Synthesis of compound 15c

3-Bromopropanoic acid (306 mg, 2.0 mmol) was dissolved in dichloromethane (10 mL), and heptadecan-9-yl 3-hydroxypropanoate 15b (658 mg, 2.0 mmol), DMAP (84 mg, 0.8 mmol), and triethylamine (248 mg, 2.4 mmol) were added. The mixture was stirred for dissolution. A solution of EDC.HCL (440 mg, 2.4 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 15c (528 mg, light yellow oil, yield: 57%).
MS m/z (ESI): 463.2 [M+1]

### Step 4: Synthesis of compound 15d

At room temperature, compound 15c (528 mg, 1.14 mmol) was dissolved in ethanol (10 mL), and butanolamine (1.5 g, 17 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 15d (480 mg, light yellow oil).
MS m/z (ESI): 472.4 [M+1]

### Step 5: Synthesis of compound 15e

3-Oxopropanoic acid (0.44 g, 5.0 mmol) was dissolved in dichloromethane (20 mL), and cholesterol (1.93 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 15a (1.03 g, light yellow oil, yield: 45%).
MS m/z (ESI): 456.4 [M+1]

### Step 6: Synthesis of compound 15f

Compound 15e (1.03 g, 2.2 mmol) was dissolved in n-butanol (10 mL), and sodium borohydride (266 mg, 7 mmol) was added at 0 °C. The mixture was stirred at room temperature for 12 h. After the starting materials were consumed completely as monitored, a saturated ammonium chloride solution was added to quench the reaction, and the resulting mixture was extracted with dichloromethane. The organic phase was dried over anhydrous sodium sulfate and concentrated to give compound 15f (980 mg, light yellow oil).
MS m/z (ESI): 459.4 [M+1]

### Step 7: Synthesis of compound 15g

3-Bromopropanoic acid (306 mg, 2.0 mmol) was dissolved in dichloromethane (10 mL), and compound 15f (916 mg, 2.0 mmol), DMAP (84 mg, 0.8 mmol), and triethylamine (248 mg, 2.4 mmol) were added. The mixture was stirred for dissolution. A solution of EDC.HCL (440 mg, 2.4 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 15g (629 mg, light yellow oil, yield: 53%).
MS m/z (ESI): 593.3 [M+1]

### Step 8: Synthesis of compound 15

Compound 15d (480 mg, 1.0 mmol) was dissolved in tetrahydrofuran, and acetonitrile, compound 15g (600 mg, 1.1 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 15 (384 mg, light yellow oil, yield: 39%).
MS m/z (ESI): 984.8 [M+1]
1H NMR (300MHz, CDCl₃): δ 5.40 (t, 1H, J = 5.4 Hz), 4.53-4.30 (m, 6H), 3.77-3.71 (m, 4H), 3.47-3.31 (m, 2H), 3.12-3.05 (m, 2H), 2.68-2.60 (m, 8H), 2.37-2.25 (m, 6H), 2.10-0.85 (m, 72H), 0.70 (s, 3H)

### Example 16. Synthesis of Compound 16

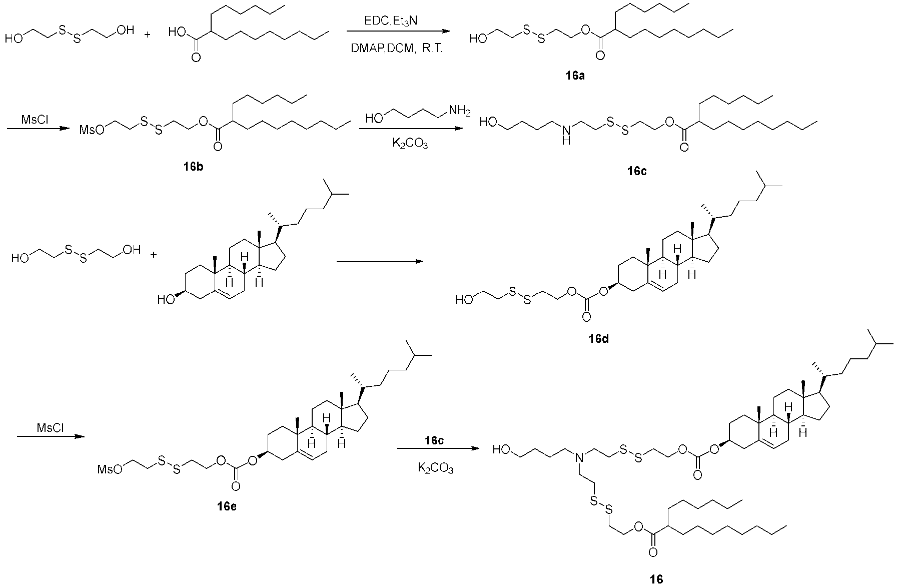

### Step 1: Synthesis of compound 16a

2-Hexyldecanoic acid (1.28 g, 5.0 mmol) was dissolved in dichloromethane (20 mL), and bis(2-hydroxyethyl)disulfide (1.54 g, 10.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated, and the residue was subjected to column chromatography to give compound 16a (1.00 g, light yellow oil, yield: 51%).
MS m/z (ESI): 393.2 [M+1]

### Step 2: Synthesis of compound 16b

Compound 16a (1.00 g, 2.5 mmol) was dissolved in dichloromethane (20 mL), and triethylamine (0.76 g, 7.5 mmol) was added. The reaction mixture was cooled to 0 °C in an ice-water bath, followed by addition of methanesulfonyl chloride (0.72 g, 6.2 mmol). The resulting mixture was stirred at room temperature for 16-20 h. After the reaction was completed, the mixture was washed twice with saturated sodium bicarbonate and washed twice with water. The organic phase was dried over anhydrous sodium sulfate, filtered, and concentrated to give compound 16b (1.00 g, light yellow oil, yield: 85%).
MS m/z (ESI): 471.2 [M+1]

### Step 3: Synthesis of compound 16c

Compound 16b (1.00 g, 2.0 mmol) was dissolved in acetonitrile (20 mL), and potassium carbonate (0.83 g, 6.0 mmol) and 4-aminobutanol (0.18 g, 2.0 mmol) were added. The mixture was stirred at room temperature for 16-20 h. After the reaction was completed, the mixture was filtered through celite. Dichloromethane was added to the filtrate, and the resulting mixture was washed twice with saturated sodium bicarbonate and washed twice with water. The organic phase was dried over anhydrous sodium sulfate, filtered, and concentrated to give compound 16c (658 mg, light yellow oil, yield: 71%).
MS m/z (ESI): 464.3 [M+1]

### Step 4: Synthesis of compound 16d

Cholesterol (1.93 g, 5.0 mmol) was dissolved in dichloromethane (20 mL), 4-dimethylaminopyridine (1.22 g, 10 mmol) was added, and then 4-nitrophenyl chloroformate (1.11 g, 5.5 mmol) was added in portions. The mixture was stirred and reacted at room temperature for 3 h. Bis(2-hydroxyethyl) disulfide (0.86 g, 5.6 mmol) was added to the reaction solution, and the resulting mixture was stirred at room temperature overnight. After the reaction was completed as detected by TLC, dichloromethane (20 mL) was added for dilution, and then the resulting mixture was washed with saturated brine (30 mL). The organic phase was dried over anhydrous sodium sulfate, filtered, and concentrated to give product 16d (1.45 g, light yellow oil, yield: 51%).
MS m/z (ESI): 567.4 [M+1]

### Step 5: Synthesis of compound 16e

Compound 16d (1.42 g, 2.5 mmol) was dissolved in dichloromethane (20 mL), and triethylamine (0.76 g, 7.5 mmol) was added. The reaction mixture was cooled to 0 °C in an ice-water bath, followed by addition of methanesulfonyl chloride (0.72 g, 6.2 mmol). The resulting mixture was stirred at room temperature for 16-20 h. After the reaction was completed, the mixture was washed twice with saturated sodium bicarbonate and washed twice with water. The organic phase was dried over anhydrous sodium sulfate, filtered, and concentrated to give compound 16e (1.26 g, light yellow oil, yield: 78%).
MS m/z (ESI): 645.3 [M+1]

### Step 6: Synthesis of compound 16

Compound 16e (1.26 g, 2.0 mmol) was dissolved in acetonitrile (20 mL), and potassium carbonate (0.83 g, 6.0 mmol) and compound 16c (0.93 g, 2.0 mmol) were added. The mixture was stirred at room temperature for 16-20 h. After the reaction was completed, the mixture was filtered through celite. Dichloromethane was added to the filtrate, and the resulting mixture was washed twice with saturated sodium bicarbonate and washed twice with water. The organic phase was dried over anhydrous sodium sulfate, filtered, and concentrated, and the residue was subjected to column chromatography to give compound 16 (871 mg, light yellow oil, yield: 43%).
MS m/z (ESI): 1012.7 [M+1]
1H NMR (300MHz, CDCl₃): δ 5.40 (t, 1H, J = 5.4 Hz), 4.53-4.40 (m, 1H), 4.20-4.02 (m, 4H), 3.67-3.61 (m, 2H), 3.58-3.50 (m, 2H), 2.47-2.42 (m, 8H), 2.03-1.72 (m, 5H), 1.67-0.85 (m, 74H), 0.70 (s, 3H)

### Example 17. Synthesis of Compound 17

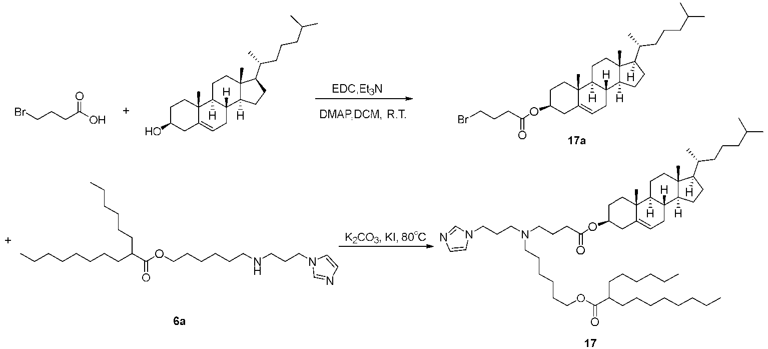

### Step 1: Synthesis of compound 17a

4-Bromobutyric acid (0.84 g, 5.0 mmol) was dissolved in dichloromethane (30 mL), and cholesterol (1.94 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 17a (1.68 g, light yellow oil, yield: 63%).
MS m/z (ESI): 535.3 [M+1]

### Step 2: Synthesis of compound 17

Compound 6a (463 mg, 1.0 mmol) was dissolved in tetrahydrofuran, and acetonitrile, compound 17a (643 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 17 (505 mg, light yellow oil, yield: 55%).
MS m/z (ESI): 918.9 [M+1]
1H NMR (300MHz, CDCl₃): δ 7.49 (s, 1H), 7.08 (s, 1H), 6.94 (s, 1H), 5.40 (t, 1H, J = 5.4 Hz), 4.53-4.40 (m, 1H), 4.10-3.92 (m, 4H), 2.48-2.25 (m, 11H), 2.10-0.85 (m, 80H), 0.70 (s, 3H)

### Example 18. Synthesis of Compound 18

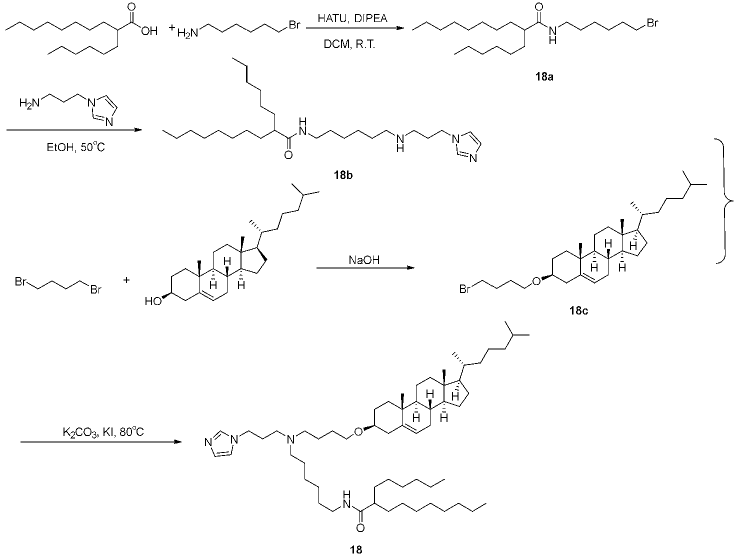

### Step 1: Synthesis of compound 18a

2-Hexyldecanoic acid (2.12 g, 5.0 mmol) was dissolved in dichloromethane (30 mL), and 6-bromohexylamine hydrochloride (1.09 g, 5.0 mmol), HATU (2.28 g, 6.0 mmol), and DIPEA (1.29 g, 10.0 mmol) were added. The mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 18a (1.40 g, light yellow oil, yield: 67%).
MS m/z (ESI): 418.3 [M+1]

### Step 2: Synthesis of compound 18b

At room temperature, compound 18a (1.25 g, 3 mmol) was dissolved in ethanol (20 mL), and 1-(3-aminopropyl)imidazole (5.63 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 18b (1.42 g, light yellow oil).
MS m/z (ESI): 463.4 [M+1]

### Step 3: Synthesis of compound 18c

1,4-Dibromobutane (2.16 g, 10.0 mmol) was dissolved in n-butanol (30 mL), and cholesterol (1.94 g, 5.0 mmol) and sodium hydroxide (1.20 g, 30 mmol) were added. The mixture was heated to reflux and stirred for 5 h. After the reaction was completed, diluted hydrochloric acid was added to adjust the pH to neutral. n-Butanol was removed by rotary evaporation, and dichloromethane (20 mL) was added for dilution. The resulting mixture was then washed with saturated brine (30 mL). The organic phase was dried over anhydrous sodium sulfate, filtered, and concentrated, and the residue was subjected to column chromatography to give product 18c (1.25 g, light yellow oil, yield: 48%).
MS m/z (ESI): 521.3 [M+1]

### Step 4: Synthesis of compound 18

Compound 18b (463 mg, 1.0 mmol) was dissolved in tetrahydrofuran, and acetonitrile, compound 18c (625 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give product 18 (434 mg, light yellow oil, yield: 48%).
MS m/z (ESI): 903.9 [M+1]
1H NMR (300MHz, CDCl₃): δ 8.01 (s, 1H), 7.49 (s, 1H), 7.08 (s, 1H), 6.94 (s, 1H), 5.40 (t, 1H, J = 5.4 Hz), 4.10-3.92 (m, 2H), 3.35 (t, 2H, J = 5.4 Hz), 3.28-3.01 (m, 7H), 2.50-2.42 (m, 4H), 2.10-0.85 (m, 83H), 0.70 (s, 3H)

### Example 19. Synthesis of Compound 19

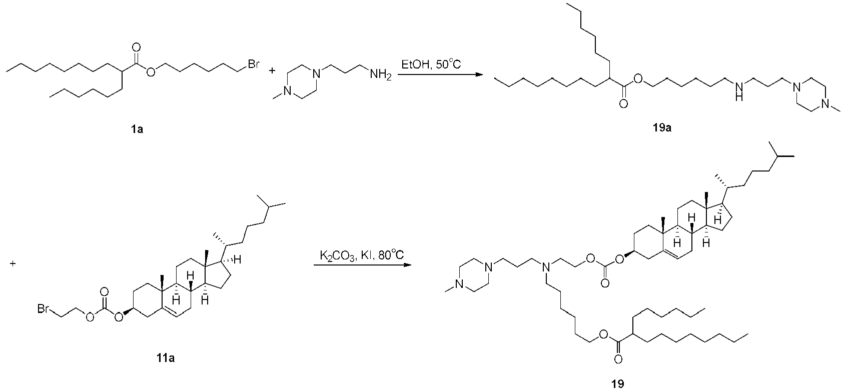

### Step 1: Synthesis of compound 19a

At room temperature, 6-bromohexyl 2-hexyldecanoate 1a (1.28 g, 3 mmol) was dissolved in ethanol (20 mL), and 1-(3-aminopropyl)-4-methylpiperazine (7.07 g, 45 mmol) was added. The mixture was heated to 50 °C and stirred for 8 h. After the starting materials were consumed completely as monitored, the reaction mixture was cooled to room temperature, and ethanol was removed at 45 °C. The crude product was dissolved in dichloromethane, and the mixture was washed three times with saturated brine. The organic phase was dried over anhydrous sodium sulfate and concentrated to give compound 19a (1.49 g, light yellow oil).
MS m/z (ESI): 496.5 [M+1]

### Step 2: Synthesis of compound 19

Compound 19a (496 mg, 1.0 mmol) was dissolved in tetrahydrofuran, and acetonitrile, compound 11a (646 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 19 (543 mg, light yellow oil, yield: 57%).
MS m/z (ESI): 952.9 [M+1]
1H NMR (300MHz, CDCl₃): δ 5.40 (t, 1H, J = 5.4 Hz), 4.53-4.40 (m, 1H), 4.20-3.95 (m, 6H), 2.74-2.65 (m, 2H), 2.47-2.40 (m, 6H), 2.36-0.85 (m, 90H), 0.70 (s, 3H)

### Example 20. Synthesis of Compound 20

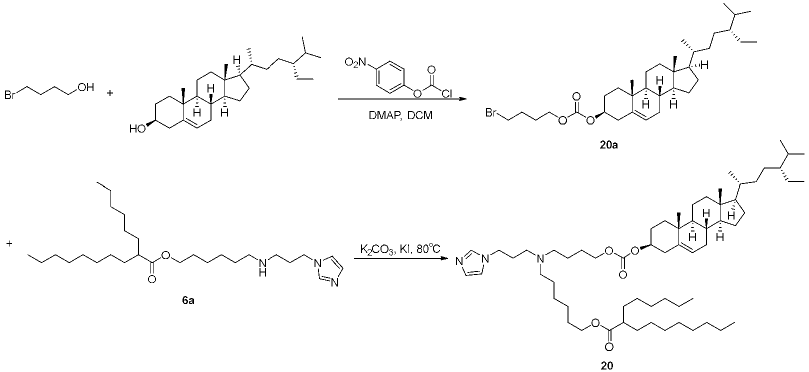

### Step 1: Synthesis of compound 20a

4-Bromobutanol (0.77 g, 5.0 mmol) was dissolved in dichloromethane (30 mL), 4-dimethylaminopyridine (1.22 g, 10 mmol) was added, and then 4-nitrophenyl chloroformate (1.11 g, 5.5 mmol) was added in portions. The mixture was stirred and reacted at room temperature for 3 h. β-Sitosterol (2.32 g, 5.6 mmol) was added to the reaction solution, and the resulting mixture was stirred at room temperature overnight. After the reaction was completed as detected by TLC, dichloromethane (20 mL) was added for dilution, and then the resulting mixture was washed with saturated brine (30 mL). The organic phase was dried over anhydrous sodium sulfate, filtered, and concentrated to give product 20a (1.63 g, light yellow oil, yield: 55%).
MS m/z (ESI): 593.3 [M+1]

### Step 2: Synthesis of compound 20

6-((3-(1H-Imidazol-1-yl)propyl)amino)hexyl 2-hexyldecanoate 6a (463 mg, 1.0 mmol) was dissolved in tetrahydrofuran, and acetonitrile, compound 20a (713 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 20 (547 mg, light yellow oil, yield: 56%).
MS m/z (ESI): 976.9 [M+1]
1H NMR (300MHz, CDCl₃): δ 7.49 (s, 1H), 7.08 (d, 1H, J = 5.6 Hz), 6.93 (d, 1H, J = 5.6 Hz), 5.40 (t, 1H, J = 5.4 Hz), 4.53-4.40 (m, 1H), 4.20-3.95 (m, 6H), 2.47-2.30 (m, 8H), 2.36-0.85 (m, 87H), 0.70 (s, 3H)

### Example 21. Synthesis of Compound 21

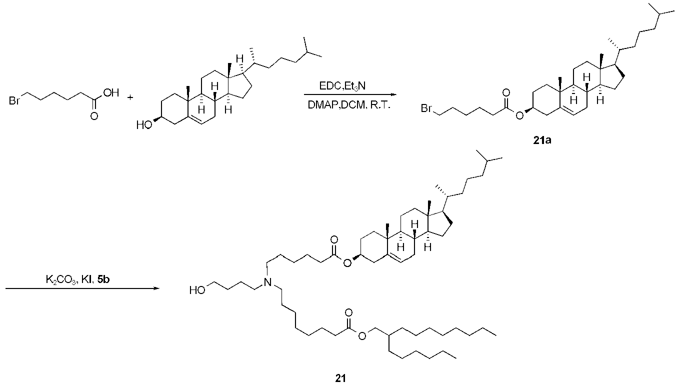

### Step 1: Synthesis of compound 21a

6-Bromohexanoic acid (0.975 g, 5.0 mmol) was dissolved in dichloromethane (30 mL), and cholesterol (1.93 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound **21a** (1.92 g, light yellow oil, yield: 68%).
MS m/z (ESI): 563.4 [M+1]

### Step 2: Synthesis of compound 21

2-Hexyldecyl 8-((4-hydroxybutyl)amino)octanoate **5b** (455 mg, 1.0 mmol) was dissolved in tetrahydrofuran, and acetonitrile, cholesterol 6-bromohexanoate **21a** (676 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound **21** (422 mg, light yellow oil, yield: 45%).
MS m/z (ESI): 938.9 [M+1]
1H NMR (400 MHz, CDCl₃ ) δ 5.27 (tt, J = 12.5, 2.0 Hz, 1H), 4.64 (p, J = 14.8 Hz, 1H), 4.31 (ddd, J = 103.0, 24.8, 14.1 Hz, 2H), 3.46 (t, J = 14.8 Hz, 2H), 2.78 - 2.63 (m, 6H), 2.44 - 2.27 (m, 4H), 2.27 - 1.47 (m, 21H), 1.45 - 1.15 (m, 47H), 1.15 - 0.96 (m, 4H), 0.95 - 0.83 (m, 21H).

### Example 22. Synthesis of Compound 24

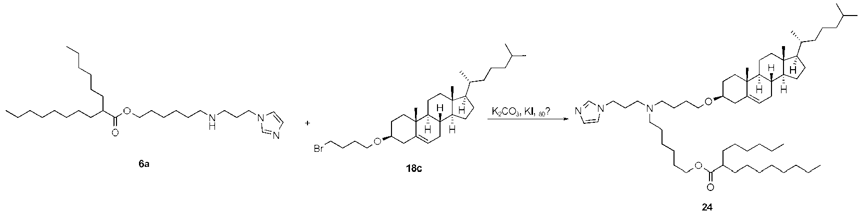

Compound 6a (463 mg, 1.0 mmol) was dissolved in tetrahydrofuran, and acetonitrile, compound 18c (625 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give product 24 (470 mg, light yellow oil, yield: 52%).
MS m/z (ESI): 904.9 [M+1]
1H NMR (400 MHz, CDCl₃ ) δ 7.92 (s, 1H), 7.24 - 6.71 (m, 2H), 5.27 (tt, J = 12.5, 2.0 Hz, 1H), 4.17 - 3.98 (m, 4H), 3.57 - 3.31 (m, 3H), 2.79 - 2.63 (m, 4H), 2.47 - 2.38 (m, 4H), 2.37 - 1.77 (m, 7H), 1.75 - 1.00 (m, 58H), 0.96 - 0.81 (m, 21H).

### Example 23. Synthesis of Compound 25

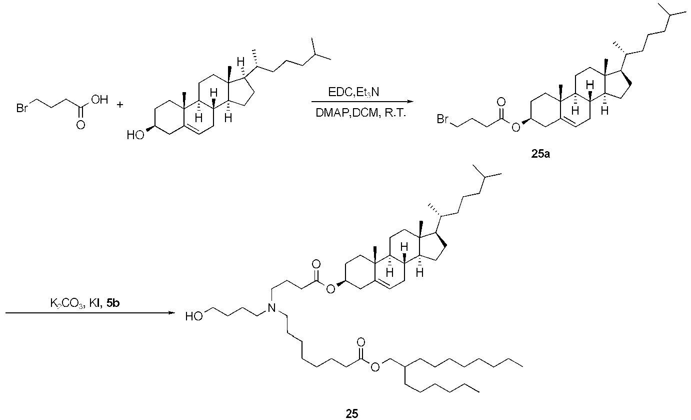

### Step 1: Synthesis of compound 25a

4-Bromobutyric acid (0.835 g, 5.0 mmol) was dissolved in dichloromethane (30 mL), and cholesterol (1.93 g, 5.0 mmol), DMAP (0.21 g, 2.0 mmol), and triethylamine (0.62 g, 6.0 mmol) were added. The mixture was stirred for dissolution. A solution of EDC.HCL (1.10 g, 6.0 mmol) in dichloromethane was added dropwise. After the dropwise addition, the mixture was stirred at room temperature for 16 h. Water was added to quench the reaction, and diluted hydrochloric acid was added to adjust the pH value to 1-3. Liquid separation was performed. The organic phase was washed with saturated brine, dried over anhydrous sodium sulfate, filtered, and concentrated to give compound 25a (1.90 g, light yellow oil, yield: 71%).
MS m/z (ESI): 535.4 [M+1]

### Step 2: Synthesis of compound 25

2-Hexyldecyl 8-((4-hydroxybutyl)amino)octanoate 5b (455 mg, 1.0 mmol) was dissolved in tetrahydrofuran, and acetonitrile, cholesterol 4-bromobutyrate 21a (643 mg, 1.2 mmol), potassium carbonate (550 mg, 4.0 mmol), and potassium iodide (332 mg, 2.0 mmol) were added. The mixture was stirred at 83 °C for 16-20 h. The reaction mixture was cooled to room temperature and filtered, and the filter residue was washed with dichloromethane. A saturated sodium bicarbonate solution was added to the obtained filtrate, and the mixture was extracted twice with dichloromethane. The organic phases were combined, dried over anhydrous sodium sulfate, filtered, and concentrated, and the residue was separated by column chromatography to give compound 25 (373 mg, light yellow oil, yield: 41%).
MS m/z (ESI): 910.9 [M+1]
1H NMR (400 MHz, CDCl₃) δ 5.27 (tt, J = 12.5, 2.0 Hz, 1H), 4.68 (p, J = 14.8 Hz, 1H), 4.31 (ddd, J = 103.0, 24.8, 13.0 Hz, 2H), 3.46 (t, J = 14.8 Hz, 2H), 2.79 - 2.62 (m, 4H), 2.43 (td, J = 12.2, 6.0 Hz, 4H), 2.37 - 2.11 (m, 3H), 2.11 - 1.80 (m, 9H), 1.79 - 1.46 (m, 11H), 1.45 - 1.15 (m, 43H), 1.14 - 0.98 (m, 4H), 0.97 - 0.80 (m, 21H).

### Example 24: mRNA- Encapsulation by Three-Component LNP Compositions and Characterization of LNP-mRNA

Taking GFP mRNA as an example, the construction of steroid-cationic lipid three-component LNP-mRNA vaccines was displayed. The lipid compound of the present disclosure, a helper lipid, and a polyethylene glycol lipid were dissolved in an ethanol solution according to the molar ratio shown in Table 1 to obtain a lipid mixture; the mRNA was dissolved in an acetic acid buffer at pH 4.0; and the lipid mixture and the mRNA were loaded onto a microfluidic device (nanoparticle preparation instrument Ignite from Precision Nanosystems) at a flow rate ratio of 1:3 to prepare an mRNA-LNP composition (see Table 1 for the specific formulation). The packaged mRNA-LNPs were dialyzed and concentrated by ultrafiltration into DPBS, and the resulting mixtures were sterilely filtered to obtain samples for subsequent animal experiments. The samples were taken and analyzed for encapsulation efficiency, average particle size, PDI, and Zeta potential.

**Table 1. Molar percentages and nitrogen-to-phosphorus ratios of lipid formulations of three-component LNPs formed from steroid-cationic lipid compounds**

| Group No. | Lipid formulation (molar percentage) | Nitrogen-to-phosphorus ratio |
|---|---|---|
| LNP1 | Compound 1:DOPE:DMG-PEG2000 = 49.25:49.25:1.5 | 6 |
| LNP2 | Compound 2:DOPE:DMG-PEG2000 = 49.25:49.25:1.5 | 4 |
| LNP3 | Compound 3:DOPE:DMG-PEG2000 = 49.25:49.25:1.5 | 8 |
| LNP4 | Compound 4:DOPE:DMG-PEG2000 = 49.25:49.25:1.5 | 6 |
| LNP5 | Compound 5:DOPE:DMG-PEG2000 = 49.25:49.25:1.5 | 6 |
| LNP6 | Compound 6:DOPE:DMG-PEG2000 = 49.25:49.25:1.5 | 8 |
| LNP7 | Compound 7:DOPE:DMG-PEG2000 = 49.25:49.25:1.5 | 6 |
| LNP8 | Compound 8:DOPE:DMG-PEG2000 = 49.25:49.25:1.5 | 6 |
| LNP9 | Compound 9:DOPE:DMG-PEG2000 = 49.25:49.25:1.5 | 8 |
| LNP10 | Compound 10:DOPE:DMG-PEG2000 = 49.25:49.25:1.5 | 8 |
| LNP11 | Compound 11:DOPE:DMG-PEG2000 = 49.25:49.25:1.5 | 8 |
| LNP12 | Compound 12:DOPE:DMG-PEG2000 = 49.25:49.25:1.5 | 6 |
| LNP13 | Compound 13:DOPE:DMG-PEG2000 = 49.25:49.25:1.5 | 6 |
| LNP14 | Compound 14:DOPE:DMG-PEG2000 = 49.25:49.25:1.5 | 6 |
| LNP15 | Compound 15:DOPE:DMG-PEG2000 = 49.25:49.25:1.5 | 6 |
| LNP16 | Compound 16:DOPE:DMG-PEG2000 = 49.25:49.25:1.5 | 6 |
| LNP17 | Compound 17:DOPE:DMG-PEG2000 = 49.25:49.25:1.5 | 6 |
| LNP18 | Compound 18:DOPE:DMG-PEG2000 = 49.25:49.25:1.5 | 6 |
| LNP19 | Compound 19:DOPE:DMG-PEG2000 = 49.25:49.25:1.5 | 10 |
| LNP20 | Compound 20:DOPE:DMG-PEG2000 = 49.25:49.25:1.5 | 6 |
| LNP21 | Compound 1:DSPC:DMG-PEG2000 = 73.87:24.62:1.5 | 6 |
| LNP22 | Compound 1:DSPC:DMG-PEG2000 = 65.7:32.8:1.5 | 6 |
| LNP23 | Compound 2:DOPE:DMG-PEG2000 = 73.87:24.62:1.5 | 4 |
| LNP24 | Compound 6:DSPC:DMG-PEG2000 = 49.25:49.25:1.5 | 6 |
| LNP25 | Compound 6:DOPE:DMG-PEG2000 = 65:33:2 | 6 |
| LNP26 | Compound 10:DOPE:DMG-PEG2000 = 47.25:47.25:5 | 8 |
| LNP27 | Compound 11:DSPC:DMG-PEG2000 = 49.25:49.25:1.5 | 8 |
| LNP28 | Compound 11:DOPE:DMG-PEG2000 = 65:33:2 | 8 |
| Control group 1 | Compound (1) of patent CN112424214A:DOPE:DMG-PEG2000 = 49.25:49.25:1.5 | 6 |
| Control group 2 | Compound (2) of patent CN112424214A:DOPE:DMG-PEG2000 = 49.25:49.25:1.5 | 6 |
| Control group 3 | Compound (3) of patent CN112424214A:DOPE:DMG-PEG2000 = 49.25:49.25:1.5 | 6 |
| Control group 4 | Compound of patent US7514099B2 (CLinDMA): DOPE:DMG-PEG2000 = 49.25:49.25:1.5 | 6 |
| Control group 5 | ALC-0315:DSPC:cholesterol:DMG-PEG2000 = 50:10:38.5:1.5 | 6 |

Among them, compound (1) of patent CN112424214A:

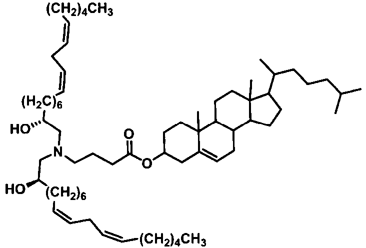

Compound (2) of patent CN112424214A:

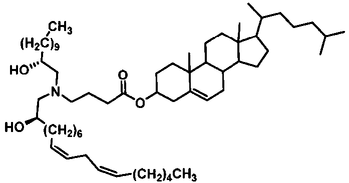

Compound (3) of patent CN112424214A:

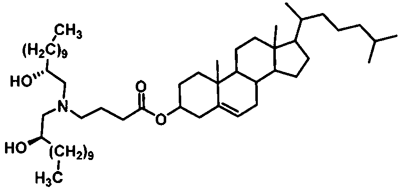

Compound of patent US7514099B2 (CLinDMA):

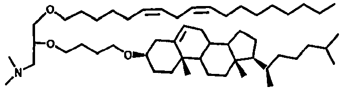

As shown in Table 2, the results show that the compositions formed from the steroid-cationic lipids of the present disclosure with the helper phosphate esters, the polyethylene glycol lipid, and the mRNA had relatively good physicochemical properties. For example, the average particle sizes were between 60 nm and 100 nm; the PDIs were all lower than 0.2, indicating relatively good polydispersity indexes; the zeta potentials were between -10 mV and +10 mV, indicating relatively weak electric properties; and the encapsulation efficiencies were all greater than 85%. The PDIs and mRNA encapsulation efficiencies of the three-component lipid nanoparticle formulations provided in the present disclosure were all superior to those of the lipid nanoparticle formulations prepared from the lipid compounds provided in CN112424214A and US7514099B2. The mRNA encapsulation efficiencies of LNPs prepared from compounds with branched side chains were higher than those of LNPs (LNP7 and LNP8) prepared from compounds with linear side chains. This demonstrates that the three-component LNPs prepared from the steroid-cationic lipid compounds provided in the present disclosure can achieve efficient encapsulation of mRNA.

**Table 2. Parameter characterization of three-component GFP-mRNA-LNP compositions formed from steroid-cationic lipid compounds**

| Sample | Average particle size (nm) | PDI | Zeta potential (mV) | Encapsulation efficiency (%) |
|---|---|---|---|---|
| LNP1 | 84.16 | 0.0950 | -0.6080 | 98 |
| LNP2 | 101.82 | 0.1805 | 0.4262 | 85 |
| LNP3 | 75.95 | 0.1208 | 1.017 | 93 |
| LNP4 | 67.47 | 0.1301 | -4.773 | 95 |
| LNP5 | 73.13 | 0.1118 | 3.376 | 90 |
| LNP6 | 60.56 | 0.1955 | 3.213 | 97 |
| LNP7 | 75.70 | 0.2175 | -9.268 | 80 |
| LNP8 | 61.97 | 0.2181 | -0.4146 | 81 |
| LNP9 | 72.86 | 0.1925 | -4.955 | 89 |
| LNP10 | 73.43 | 0.1345 | -0.4146 | 89 |
| LNP11 | 86.8 | 0.0805 | -3.400 | 91 |
| LNP12 | 93.62 | 0.1175 | -6.023 | 88 |
| LNP13 | 64.07 | 0.1645 | 1.692 | 89 |
| LNP14 | 73.43 | 0.1345 | -0.4146 | 89 |
| LNP15 | 81.68 | 0.1087 | -4.955 | 87 |
| LNP16 | 70.23 | 0.1483 | 0.8040 | 95 |
| LNP17 | 67.47 | 0.1638 | 5.283 | 97 |
| LNP18 | 75.95 | 0.0947 | -0.3641 | 94 |
| LNP19 | 75.25 | 0.0832 | -7.539 | 93 |
| LNP20 | 92.76 | 0.1104 | -2.188 | 93 |
| LNP21 | 72.36 | 0.1523 | 4.382 | 92 |
| LNP22 | 55.89 | 0.1850 | 14.40 | 99 |
| LNP23 | 66.16 | 0.1923 | 1.859 | 86 |
| LNP24 | 64.83 | 0.1667 | 0.7289 | 87 |
| LNP25 | 60.56 | 0.1481 | 3.213 | 97 |
| LNP26 | 65.27 | 0.1756 | 2.656 | 86 |
| LNP27 | 58.27 | 0.1807 | 12.53 | 99 |
| LNP28 | 95.91 | 0.1555 | 0.4262 | 91 |
| Control group 1 | 123.30 | 0.2334 | 3.269 | 78 |
| Control group 2 | 138.27 | 0.2150 | 3.564 | 79 |
| Control group 3 | 89.93 | 0.1938 | -8.152 | 78 |
| Control group 4 | 141.30 | 0.2226 | -2.365 | 77 |
| Control group 5 | 73.13 | 0.0486 | -3.222 | 92 |

### Example 25: Cell Assay for GFP-Encoding mRNA-LNP Compositions

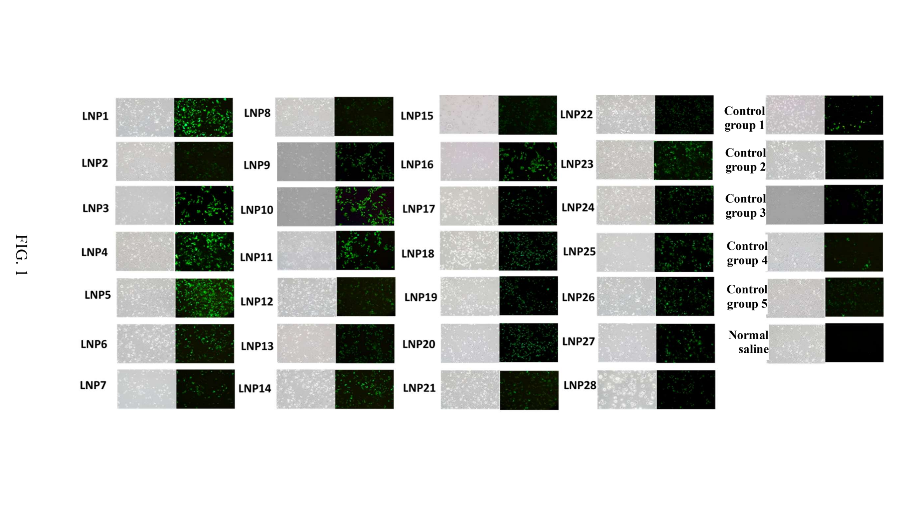

Hep3B cells at a cell density of 6.5 × 10⁵ cells/mL were seeded into a 24-well cell culture plate at 1 mL/well. 24 h later, the cells in each well were transfected with 500 ng of GFP mRNA-LNP (prepared in Example 24). The cell culture plate was then placed in a cell incubator at 37 °C with 5% CO₂ for culture. The negative control group was transfected with an equal volume of normal saline. After 24 hours, the microscopic images were taken. The results are shown in FIG. 1. The results show that the three-component LNP compositions formed by using the steroid-cationic lipid compounds of the present disclosure could achieve high expression of GFP-mRNA in cells, and the expression level was significantly higher than that of the control groups. Among them, the encapsulation and delivery efficiencies of the LNPs formed from the compounds in which the aliphatic chain structure was a branched structure for mRNA were higher than those of the compounds in which the aliphatic chain structure was a linear structure, which may be because this branched structure, as a lipid layer, is more easily converted from a lamellar phase to an inverse hexagonal phase, thereby facilitating the release of mRNA and improving the transfection efficiency.

### Example 26: Animal Immunization Assay for SARS-CoV-2 Antigen mRNA-LNP Compositions

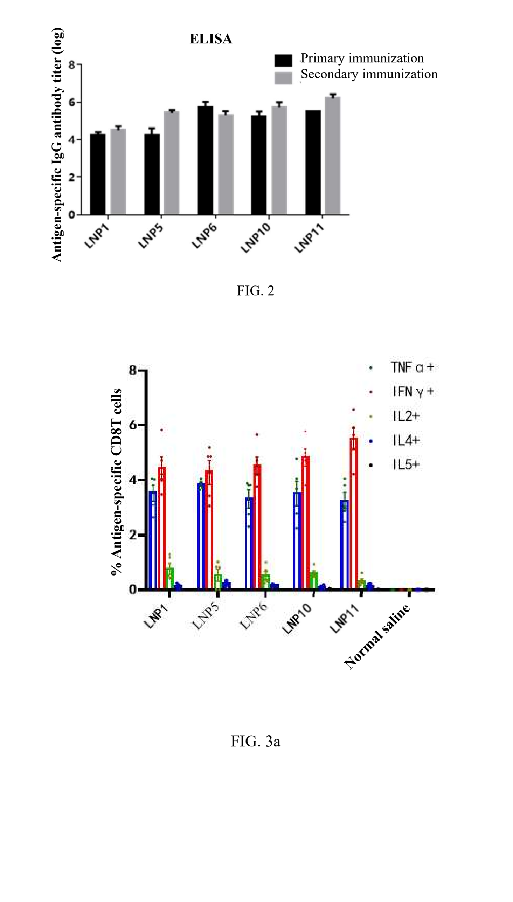

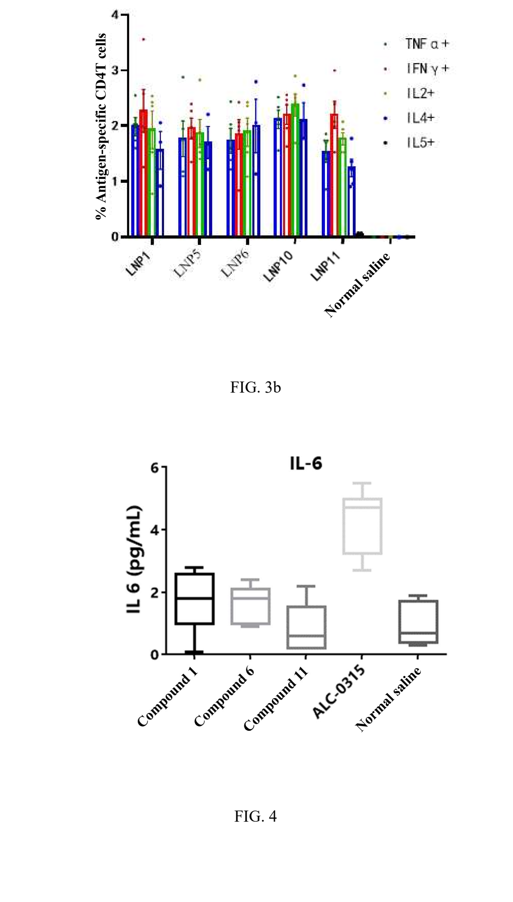

SARS-CoV-2 S protein antigen mRNA was used to prepare SARS-CoV-2 antigen mRNA-LNPs according to the lipid formulation in Table 1 of Example 24. Female BALB/c mice aged 6-8 weeks were randomly divided into groups with 5 mice per group, and immunized via intramuscular injection in the hind leg. The mice were immunized on day 0 and day 14 with an immunization dose of 3 µg of mRNA-LNPs, and the negative control group was injected with an equal volume of normal saline. Blood was collected on day 14 after immunization, and serum was isolated. The specific antibody titer against the SARS-CoV2 S protein antigen was detected by ELISA. The ICS antigen-specific cellular immunity detection was performed on day 28. The results are shown in FIGs. 2, 3a, and 3b. The LNP1, LNP5, LNP6, LNP10, and LNP11 experimental groups could all induce relatively good humoral immunity and cellular immunity (in each group in FIG. 2, the left column shows the IgG antibody titer after primary immunization, and the right column shows the IgG antibody titer after secondary immunization; in each group in FIGs. 3a and 3b, the columns show TNFα+, TNFγ+, IL2+, IL4+, and IL5+ from left to right).

### Example 27. Safety Evaluation of Cholesterol Cationic Compounds

CD1 mice were inoculated with the SARS-CoV-2 antigen mRNA-LNP complex at a dose of 30 µg by intramuscular injection, and the negative control group was injected with an equal volume of normal saline. The safety of the three-component LNPs based on compound 1, compound 6, and compound 11 was evaluated by monitoring the systemic inflammatory factor IL-6 in mice. As shown in FIG. 4, after high-dose administration, the expression levels of the systemic inflammatory factor IL-6 caused by the three-component LNP experimental groups based on compound 1, compound 6, and compound 11 were significantly reduced compared with that caused by the four-component LNP of the launched compound ALC-0315.

### Example 28. Lyophilization Study of Three-Component LNPs of Cholesterol Cationic Lipid Compounds

Taking the GFP-mRNA-LNP complex as an example, the mRNA-LNP was dissolved in a 20 mM Tris-sodium acetate buffer at pH 7.5 containing 10% sucrose by mass, and 300 µL of the resulting solution was taken and added to a 3 mL vial for lyophilization. Lyophilization procedures: pre-freezing stage: -30 °C for 2 h; desorption drying stage: 25 °C for 1 h; sublimation drying stage: -40 °C for 2 h and -20 °C for 2 h, with vacuum pressure controlled to be ≤ 10 Pa during the whole process. The sample was subjected to stability storage at 2-8 °C for 1 month. The total amount of mRNA, the concentration of free mRNA, the encapsulation efficiency, and the mRNA integrity were detected after reconstitution with sterile enzyme-free water.

**Table 3. Changes in key quality attributes of LNPs before and after lyophilization**

| Sample experimental group | | Average particle size (nm) | PDI | Z-potential (mV) | Encapsulation efficiency (%) |
|---|---|---|---|---|---|
| LNP1 | Before lyophilization | 84.16 | 0.0950 | -0.608 | 98 |
| | After lyophilization | 90.13 | 0.0980 | -2.130 | 96 |
| LNP2 | Before lyophilization | 101.82 | 0.1805 | 0.4262 | 85 |
| | After lyophilization | 110.23 | 0.1870 | -4.12 | 84 |
| LNP3 | Before lyophilization | 75.95 | 0.1208 | 1.017 | 93 |
| | After lyophilization | 84.80 | 0.1500 | -2.21 | 90 |
| LNP4 | Before lyophilization | 67.47 | 0.1301 | -4.773 | 95 |
| | After lyophilization | 80.12 | 0.1120 | -4.31 | 95 |
| LNP5 | Before lyophilization | 73.13 | 0.1118 | 3.376 | 90 |
| | After lyophilization | 83.12 | 0.1240 | 1.223 | 85 |
| LNP6 | Before lyophilization | 60.56 | 0.1955 | 3.213 | 97 |
| | After lyophilization | 75.12 | 0.1520 | -4.11 | 92 |
| LNP7 | Before lyophilization | 75.70 | 0.2175 | -9.268 | 80 |
| | After lyophilization | 72.15 | 0.0710 | -10.23 | 74 |
| LNP8 | Before lyophilization | 61.97 | 0.2181 | -0.4146 | 81 |
| | After lyophilization | 80.23 | 0.1422 | -3.78 | 75 |
| LNP9 | Before lyophilization | 72.86 | 0.1925 | -4.955 | 89 |
| | After lyophilization | 79.23 | 0.1650 | -4.83 | 85 |
| LNP10 | Before lyophilization | 73.43 | 0.1345 | -0.4146 | 89 |
| | After lyophilization | 90.13 | 0.0923 | -3.98 | 85 |
| LNP11 | Before lyophilization | 86.80 | 0.0805 | -3.4 | 91 |
| | After lyophilization | 90.12 | 0.0912 | -3.11 | 88 |
| LNP12 | Before lyophilization | 93.62 | 0.1175 | -6.023 | 88 |
| | After lyophilization | 113.40 | 0.1210 | -6.24 | 83 |
| LNP13 | Before lyophilization | 64.07 | 0.1645 | 1.692 | 89 |
| | After lyophilization | 86.77 | 0.2000 | -4.35 | 84 |
| LNP14 | Before lyophilization | 73.43 | 0.1345 | -0.4146 | 89 |
| | After lyophilization | 82.13 | 0.1025 | -0.1254 | 85 |
| LNP15 | Before lyophilization | 81.68 | 0.1087 | -4.955 | 87 |
| | After lyophilization | 90.01 | 0.0856 | 3.66 | 83 |
| LNP16 | Before lyophilization | 70.23 | 0.1483 | 0.804 | 95 |
| | After lyophilization | 78.21 | 0.1325 | 1.265 | 92 |
| LNP17 | Before lyophilization | 67.47 | 0.1638 | 5.283 | 97 |
| | After lyophilization | 75.36 | 0.1325 | 4.12 | 95 |
| LNP18 | Before lyophilization | 75.95 | 0.0947 | -0.3641 | 94 |
| | After lyophilization | 80.23 | 0.0845 | 0.0125 | 90 |
| LNP19 | Before lyophilization | 75.25 | 0.0832 | -7.539 | 93 |
| | After lyophilization | 83.35 | 0.1036 | -3.216 | 89 |
| LNP20 | Before lyophilization | 92.76 | 0.1104 | -2.188 | 93 |
| | After lyophilization | 100.20 | 0.1202 | 1.255 | 90 |
| LNP21 | Before lyophilization | 72.36 | 0.1523 | 4.382 | 92 |
| | After lyophilization | 81.20 | 0.1236 | 2.565 | 90 |
| LNP22 | Before lyophilization | 55.89 | 0.1850 | 14.4 | 99 |
| | After lyophilization | 65.02 | 0.1256 | 10.32 | 95 |
| LNP23 | Before lyophilization | 66.16 | 0.1923 | 1.859 | 86 |
| | After lyophilization | 73.25 | 0.1462 | 3.654 | 81 |
| LNP24 | Before lyophilization | 64.83 | 0.1667 | 0.7289 | 87 |
| | After lyophilization | 73.26 | 0.1526 | 6.325 | 83 |
| LNP25 | Before lyophilization | 60.56 | 0.1481 | 3.213 | 97 |
| | After lyophilization | 68.36 | 0.1256 | 7.541 | 92 |
| LNP26 | Before lyophilization | 65.27 | 0.1756 | 2.656 | 86 |
| | After lyophilization | 73.84 | 0.1862 | 5.625 | 82 |
| LNP27 | Before lyophilization | 58.27 | 0.1807 | 12.53 | 99 |
| | After lyophilization | 66.19 | 0.1484 | 13.24 | 95 |
| LNP28 | Before lyophilization | 95.91 | 0.1555 | 0.4262 | 91 |
| | After lyophilization | 103.75 | 0.1654 | 3.785 | 88 |
| Control group 1 | Before lyophilization | 123.30 | 0.2334 | 3.269 | 78 |
| | After lyophilization | 140.35 | 0.2126 | 7.364 | 71 |
| Control group 2 | Before lyophilization | 138.27 | 0.2150 | 3.564 | 79 |
| | After lyophilization | 151.27 | 0.3080 | 7.625 | 70 |
| Control group 3 | Before lyophilization | 89.93 | 0.1938 | -8.152 | 78 |
| | After lyophilization | 112.36 | 0.2322 | 0.2562 | 71 |
| Control group 4 | Before lyophilization | 141.30 | 0.2226 | -2.365 | 77 |
| | After lyophilization | 136.90 | 0.1792 | 3.185 | 70 |
| Control group 5 | Before lyophilization | 73.13 | 0.0486 | -3.222 | 92 |
| | After lyophilization | 109.55 | 0.1560 | 3.611 | 63 |

The results are shown in Table 3. The stability (e.g., particle size, PDI, and encapsulation efficiency parameters) of the three-component LNPs prepared from the lipid compounds provided in the present disclosure after lyophilization was significantly higher than that of the control groups. The LNPs prepared from the lipid compounds provided in the present disclosure had particle size changes of less than 10 nm before and after lyophilization, and the encapsulation efficiency changes were all within 6%; the particle sizes of the LNPs in the control groups increased by more than 10 nm after lyophilization, and the mRNA encapsulation efficiency changes were all greater than 10%.

### Example 29. Nebulization Study of Three-Component LNPs of Cholesterol Cationic Lipid Compounds

GFP-mRNA-LNP complexes were used. mRNA-LNPs were nebulized by a nebulizer, and then droplets were collected. The particle size, PDI, and electric potential were detected using a nanoparticle size detector, and the content and encapsulation efficiency were detected using a Ribogreen detection kit.

**Table 4. Key quality attribute parameters before and after LNP nebulization**

| Sample experimental group | | Average particle size (nm) | PDI | Z-potential (mV) | Encapsulation efficiency (%) |
|---|---|---|---|---|---|
| LNP1 | Before nebulization | 84.16 | 0.0950 | -0.608 | 98 |
| | After nebulization | 154.73 | 0.2077 | -11.67 | 94 |
| LNP4 | Before nebulization | 67.47 | 0.1301 | -4.773 | 95 |
| | After nebulization | 156.82 | 0.2130 | -9.132 | 94 |
| LNP5 | Before nebulization | 73.13 | 0.1118 | 3.376 | 90 |
| | After nebulization | 163.90 | 0.1868 | -7.954 | 90 |
| LNP10 | Before nebulization | 73.43 | 0.1345 | -0.4146 | 89 |
| | After nebulization | 144.66 | 0.2000 | -3.856 | 81 |
| Control group 3 | Before nebulization | 89.93 | 0.1938 | -8.152 | 78 |
| | After nebulization | 190.38 | 0.2353 | -20.13 | 48 |
| Control group 4 | Before nebulization | 141.30 | 0.2226 | -2.365 | 77 |
| | After nebulization | 207.55 | 0.3927 | -15.57 | 38 |
| Control group 5 | Before nebulization | 73.13 | 0.0486 | -3.222 | 92 |
| | After nebulization | 239.43 | 0.5013 | -25.71 | Not detected |

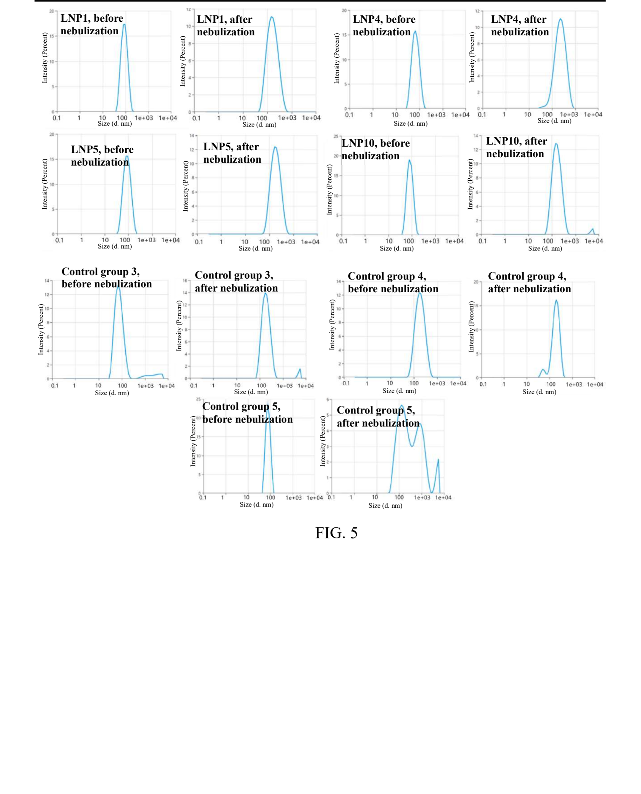

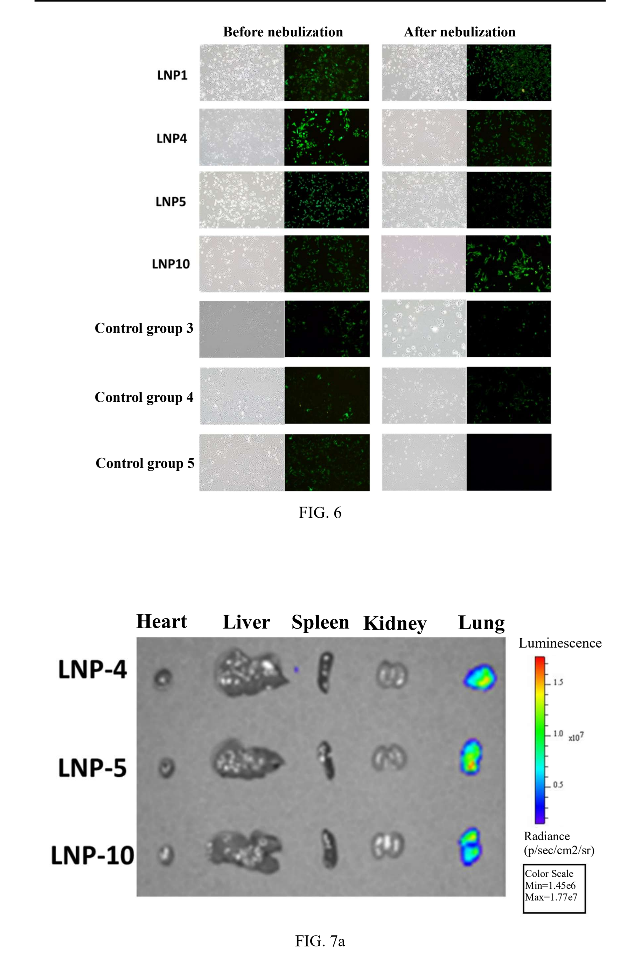

The experimental results are shown in Table 4 and FIG. 5. After the LNP1, LNP4, LNP5, and LNP10 samples were nebulized, the key quality attributes such as particle size, PDI, and encapsulation efficiency exhibited little change; and the samples still maintained a relatively good particle state, and the encapsulation efficiency change was also less than 10% after nebulization. In comparison, the particle sizes of the LNP formulations in the control groups 3 and 4 were close to 200 nm after nebulization, and the encapsulation efficiencies also decreased to nearly 40%. The particle structure of the four-component LNP based on ALC-0315 in the control group 5 was damaged after nebulization, and the encapsulation efficiency was even undetectable. The samples after nebulization were transfected into Hep3B cells at 500 ng mRNA-LNP/10⁵ cells/well, and the expression level of GFP was detected by microscopic imaging after 24 h. The results are shown in FIG. 6. The cells treated with the samples of the LNP formulations in the control groups 3 and 4 after nebulization exhibited relatively weak expression levels, and the four groups of samples LNP1, LNP4, LNP5, and LNP10 were still able to retain relatively good cell transfection activity after nebulization, whereas no cells were expressed in the four-component LNP group after nebulization.

### Example 30. Expression in Lungs of Mice after Aerosol Inhalation of LNPs

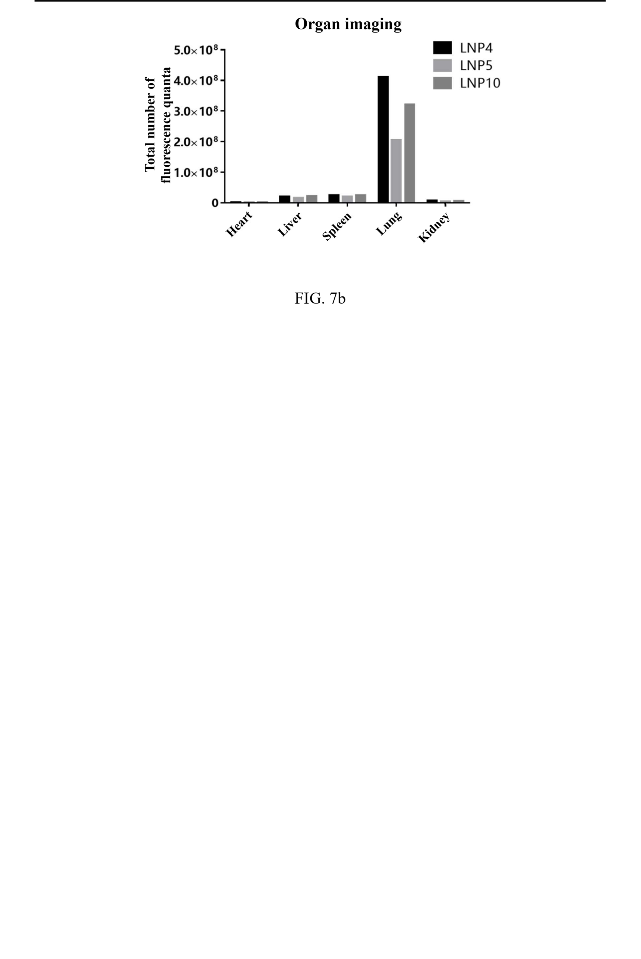

Following the procedures in Example 24, Luc mRNA was encapsulated using the LNP4, LNP5, and LNP10 formulations. C57BL/6 female mice aged 7-9 weeks were selected and received 10 µg of Luc-mRNA-LNP complexes by nebulization. Six hours after administration, the mice were anesthetized and then injected intraperitoneally with a fluorescent substrate. The mice were sacrificed and dissected, and fluorescence imaging was performed on the heart, liver, spleen, lung, and kidney. The total number of fluorescence quanta was counted. The results are shown in FIGs. 7a and 7b (in each group in FIG. 7b, the columns show LNP4, LNP5, and LNP10 from left to right). After the mice were given Luc-mRNA-LNPs by aerosol inhalation, very strong fluorescence expression could be detected in the lungs. This indicates that after aerosol inhalation of LNP4, LNP5 and LNP10, the mRNA was able to be selectively expressed in the lungs of mice, thereby further suggesting that the three-component LNPs based on the cholesterol cationic lipid compounds designed therein can be used in applications of aerosol inhalation mRNA treatment.

Finally, it should be noted that, the above examples are only used to illustrate the technical solutions of the present disclosure, but not to limit the present disclosure. Although the present disclosure is described in detail with reference to the examples described above, it will be understood by those skilled in the art that, modifications may still be made to the technical solutions in the examples described above, or equivalent substitutions may be applied to some or all of the technical features; and these modifications or substitutions do not make the essence of the corresponding technical solutions depart from the scope of the technical solutions in the examples of the present disclosure.

## Claims

1. A compound represented by formula (I) 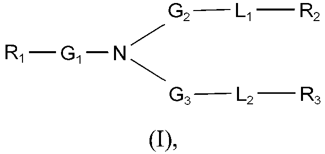
wherein R₁ is selected from -OR₄, -NR₄R₅, -NR₄C(=O)R₅, -C(=O)OR₅, -OC(=O)R₅, -OC(=O)OR₅, - CN, a nitrogen-containing heterocyclic group, and guanidino;
R₄ and R₅ are each independently selected from H, C₁₋₉ alkyl, C₂₋₉ alkenyl, C₂₋₉ alkynyl, C₃₋₈ cycloalkyl, C₃₋₈ cycloalkenyl, and C₃₋₈ cycloalkynyl;
G₁ is selected from a chemical bond (-), C₁₋₉ alkylene, C₂₋₉ alkenylene, C₃₋₉ alkynylene, C₃₋₈ cycloalkylene, and C₃₋₈ cycloalkenylene;
preferably, R₁-G₁- has no more than 10 contiguous carbon atoms;
G₂ and G₃ are each independently selected from a chemical bond (-), C₁₋₁₂ alkylene, C₂₋₁₂ alkenylene, C₂₋₁₂ alkynylene, C₃₋₁₂ cycloalkylene, C₃₋₁₂ cycloalkenylene, C₃₋₁₂ cycloalkynylene, and C₆₋₁₂ arylene; L₁ and L₂ are each independently selected from one or a combination of two or more of a chemical bond (-), -O-, -S-, -O(C=O)O-, -(C=O)NRₐ-, -NRₐ(C=O)-, -O(C=O)-, -(C=O)O-, -S-S-, -S(O)ₓ-, - OS(O)ₓO-, -C(=O)S-, -SC(=O)-, -NRₐC(=O)NR_{b}-, -OC(=O)NRₐ-, -NRₐC(=O)O-, -OC(=O)S-, - SC(=O)O-, -P(O)(ORₐ)O-, -OP(O)(ORₐ)O-, and C₁₋₁₂ alkylene;
x is selected from 0, 1, and 2;
Rₐ and R_{b} are each independently selected from H, C₁₋₁₂ alkyl, C₂₋₁₂ alkenyl, and C₂₋₁₂ alkyne;
R₂ is selected from a naturally occurring or non-naturally occurring steroid;
R₃ is selected from a naturally occurring or non-naturally occurring steroid, C₆₋₂₄ alkyl, C₆₋₂₄ alkenyl, C₆₋₂₄ alkyne, and C₆₋₂₄ alkoxy.

2. The compound according to claim 1, wherein R₁ is selected from -OR₄, -NR₄R₅, pyrazolyl, imidazolyl, piperazinyl, alkylpiperazine, piperidinyl, alkylpiperidine, guanidino, pyrrolyl, and pyrrolidinyl; R₄ and R₅ are each independently selected from H, C₁₋₉ alkyl, C₂₋₉ alkenyl, C₂₋₉ alkynyl, C₃₋₈ cycloalkyl, and C₃₋₈ cycloalkenyl;
G₁ is a chemical bond (-), C₁₋₉ alkylene, C₂₋₉ alkenylene, C₃₋₈ cycloalkylene, or C₃₋₈ cycloalkenylene; preferably, G₁ is C₁₋₆ alkylene or -C₂₋₆ alkenylene;
preferably, R₁-G₁- has no more than 10 contiguous carbon atoms;
G₂ and G₃ are each independently selected from a chemical bond (-), C₁₋₁₂ alkylene, C₂₋₁₂ alkenylene,
and -C₂₋₁₂ alkynylene;
L₁ and L₂ are each independently selected from one or a combination of two or more of -O(C=O)-, - (C=O)O-, -S-S-, -O(C=O)O-, -NRₐC(=O)O-, -(C=O)NRₐ-, -NRₐ(C=O)-, -C(=O)S-, -SC(=O)-, - OC(=O)NRₐ-, and C₁₋₁₂ alkylene;
wherein Rₐ is H, C₁₋₁₂ alkyl, C₂₋₁₂ alkenyl, or C₂₋₁₂ alkyne.

3. The compound according to claim 1, wherein R₁ is selected from -OR₄, -NR₄R₅, imidazolyl, piperazinyl, and guanidino;
R₄ and R₅ are each independently selected from H and C₁₋₅ alkyl;
G₁ is unsubstituted C₁₋₆ alkylene;
preferably, R₁-G₁- has no more than 10 contiguous carbon atoms;
G₂ and G₃ are each independently selected from a chemical bond (-) and C₁₋₁₀ alkylene;
L₁ and L₂ are each independently selected from -O-, -O(C=O)-, -O(C=O)O-, -(C=O)O-, -(C=O)S-, - NHC(=O)O-, -NHC(=O)-,

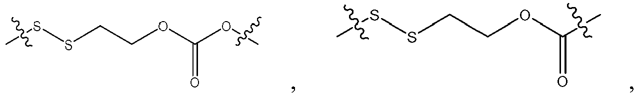

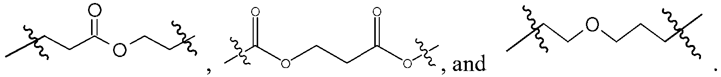

4. The compound according to claim 1, wherein R₂ is a sterol;
preferably, the sterol is a zoosterol or an oxidized or reduced form thereof; and/or the sterol is a phytosterol or an oxidized or reduced form thereof; and/or the sterol is a synthetic sterol or an oxidized or reduced form thereof;
more preferably, the sterol is selected from cholesterol, an oxidized form of cholesterol, a reduced form of cholesterol, alkyl lithocholate, stigmasterol, stigmastanol, campesterol, ergosterol, and sitosterol;
more preferably, the sterol is an oxidized form of cholesterol, a reduced form of cholesterol, alkyl lithocholate, stigmasterol, stigmastanol, campesterol, ergosterol, or sitosterol;
more preferably, the sterol is selected from avenasterol, β-sitosterol, brassicasterol, ergocalciferol, campesterol, cholestanol, cholesterol, coprosterol, dehydrocholesterol, desmosterol, dihydroergocalciferol, dihydrocholesterol, dihydroergosterol, dinosterol, epicholesterol, ergosterol, fucosterol, hexahydrolumisterol, hydroxycholesterol, lanosterol, lumisterol, saringosterol, sitostanol, sitosterol, stigmastanol, stigmasterol, cholic acid, glycocholic acid, taurocholic acid, deoxycholic acid, and lithocholic acid;
more preferably, the sterol has the following structural formula: 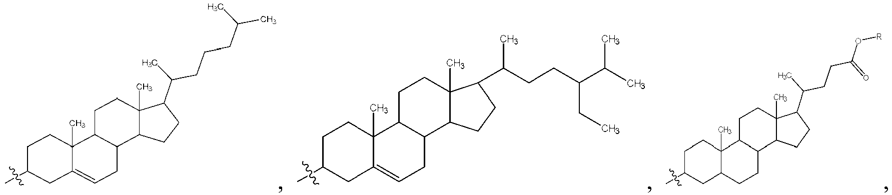 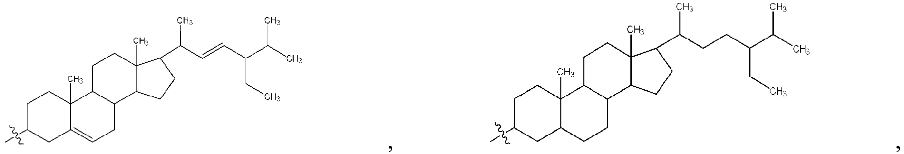 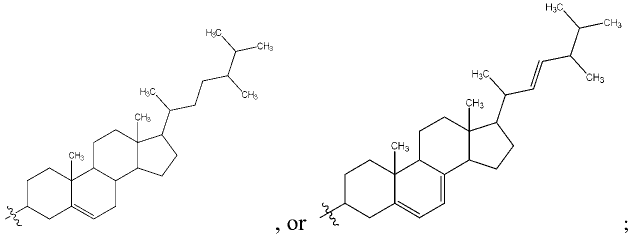
wherein R is C₁₋₁₀ alkyl.

5. The compound according to any one of claims 1-4, wherein R₃ is selected from: 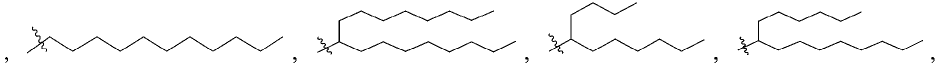 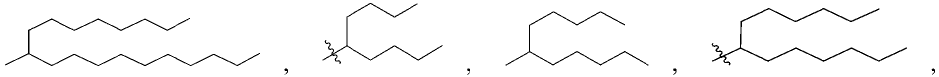 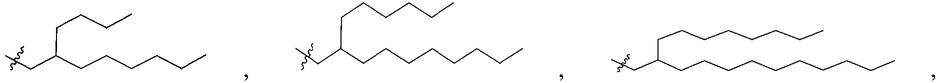 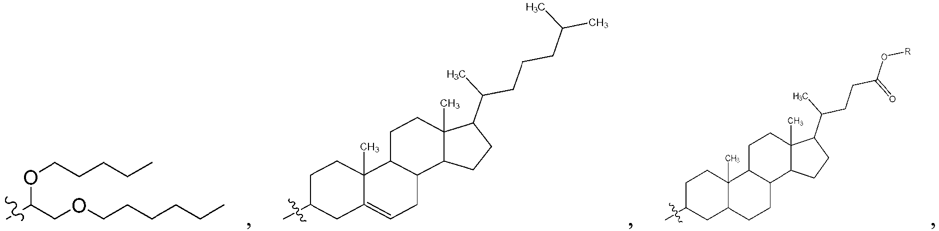 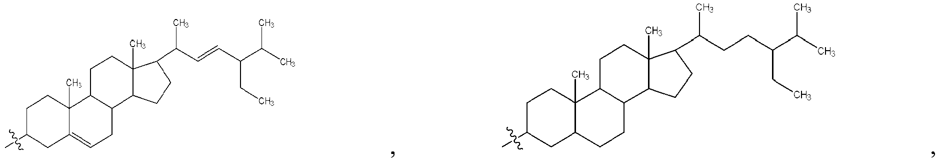 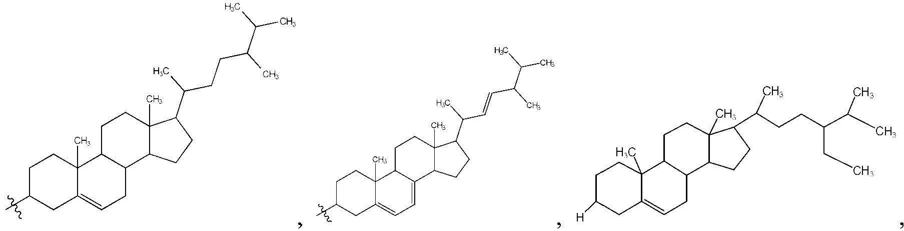 and 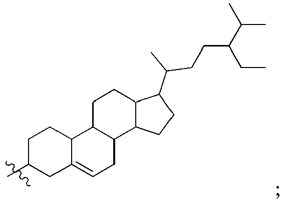 preferably, R₃ is selected from:

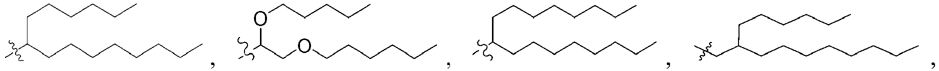

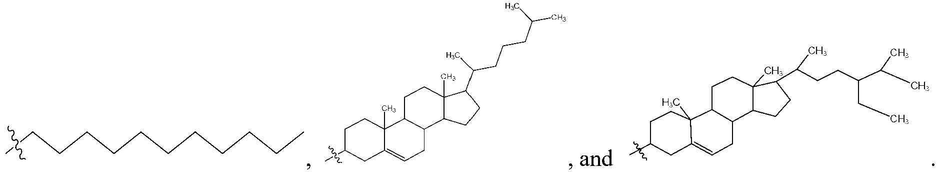

6. The compound according to any one of claims 1-5, wherein the compound is selected from: 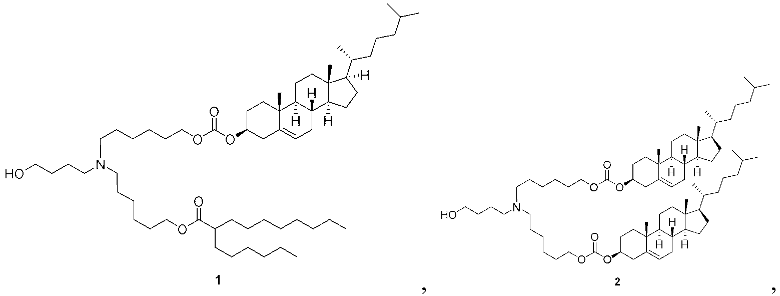 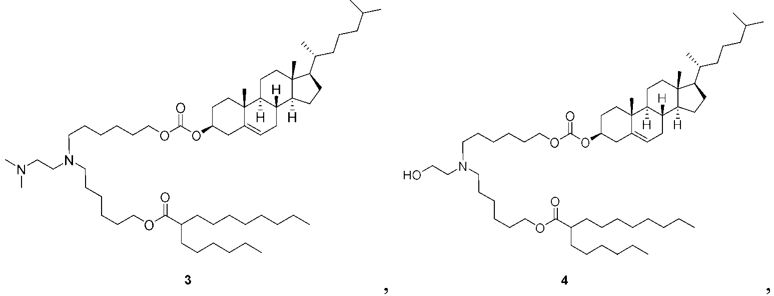 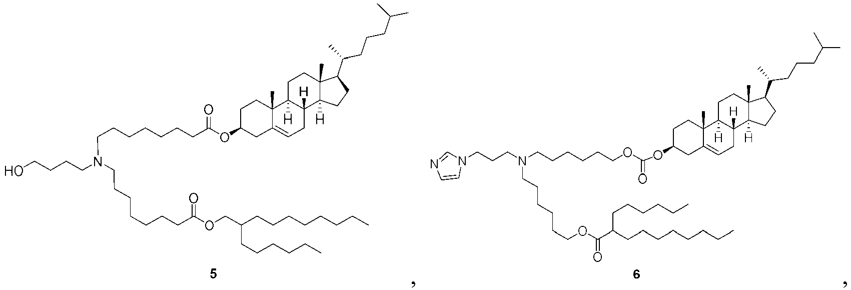 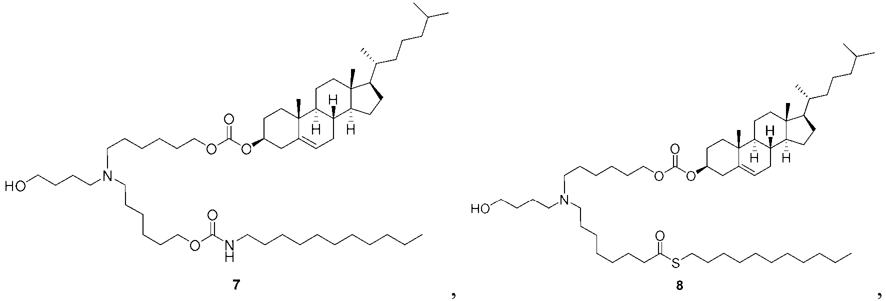 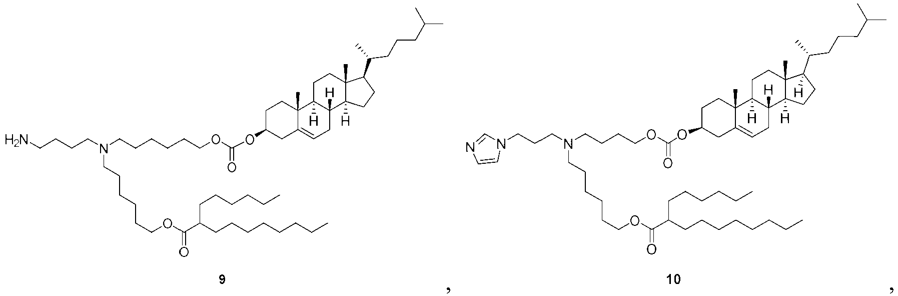 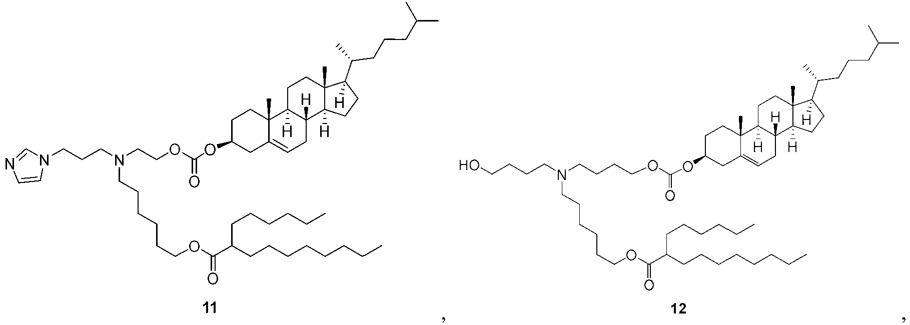 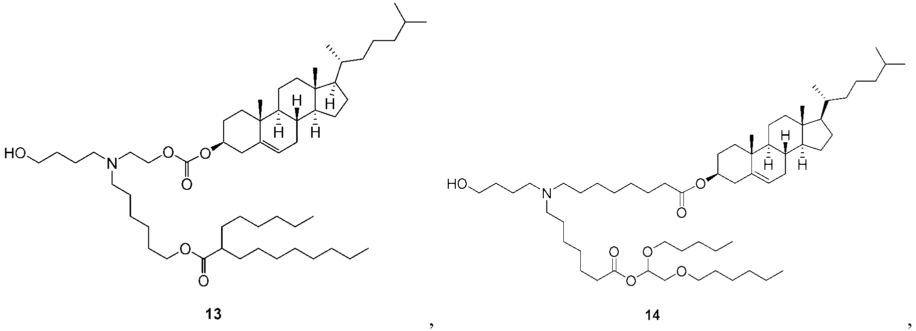 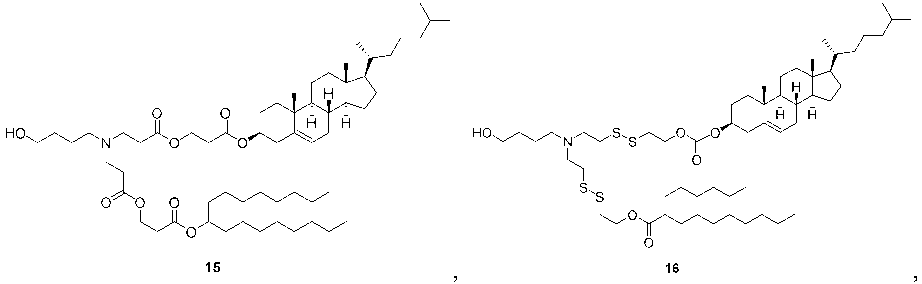 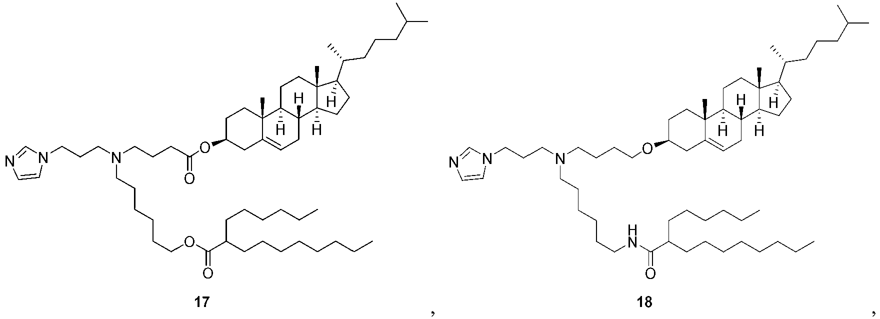 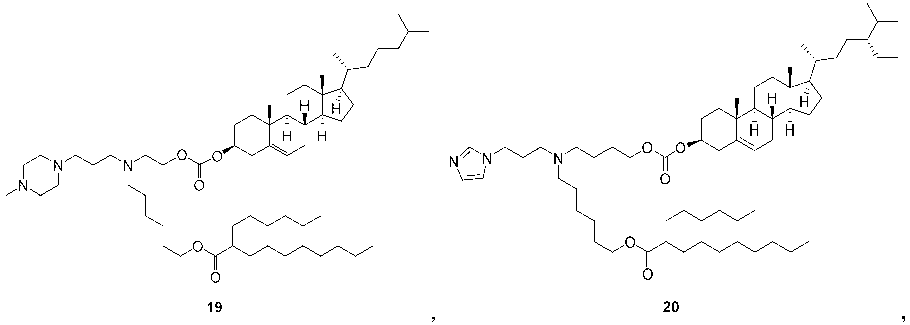 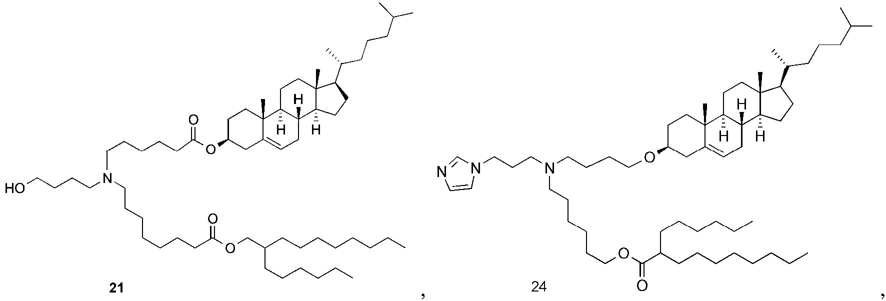 and

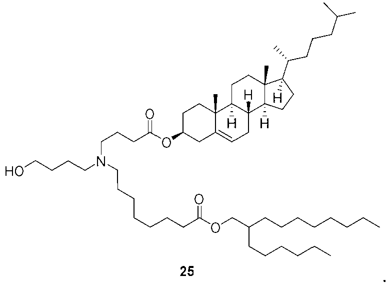

7. A lipid nanoparticle, comprising the compound according to any one of claims 1-6.

8. The lipid nanoparticle according to claim 7, further comprising a polyethylene glycol lipid and at least one helper lipid, wherein the helper lipid is selected from a neutral lipid, a zwitterionic lipid, and an anionic lipid.

9. The lipid nanoparticle according to claim 8, wherein the polyethylene glycol lipid is selected from 2-[(polyethylene glycol)-2000]-N,N-ditetradecylacetamide (ALC-0159), 1,2-dimyristoyl-sn-glycero-methoxypolyethylene glycol (PEG-DMG), 1,2-distearoyl-sn-glycero-3-phosphoethanolamine-N-[amino(polyethylene glycol)] (PEG-DSPE), PEG-disterol glycerol (PEG-DSG), PEG-dipalmitoyl, PEG-dioleyl, PEG-distearyl, PEG-diacylglycerol amide (PEG-DAG), PEG-dipalmitoyl phosphatidylethanolamine (PEG-DPPE),
and PEG-1,2-dimyristoyloxypropyl-3-amine (PEG-c-DMA);
the helper lipid is selected from 1,2-distearoyl-sn-glycero-3-phosphocholine (DSPC), 1,2-dipalmitoyl-sn-glycero-3-phosphocholine (DPPC), 1,2-dioleoyl-sn-glycero-3-phosphoethanolamine (DOPE), 1,2-dipalmitoyl-sn-glycero-3-phosphoethanolamine (DPPE), 1,2-dimyristoyl-sn-glycero-3-phosphoethanolamine (DMPE), 2-dioleoyl-sn-glycero-3-phospho-(1'-rac-glycerol) (DOPG), palmitoyloleoylphosphatidylcholine (POPC), 1-palmitoyl-2-oleoylphosphatidylethanolamine (POPE), phosphocholine (DOPC), dimyristoylphosphatidylcholine (DMPC), phosphatidylcholine (PLPC), 1,2-distearoyl-sn-glycero-3-phosphocholine (DAPC), phosphatidylethanolamine (PE), egg phosphatidylcholine (EPC), dilauroylphosphatidylcholine (DLPC), dimyristoylphosphatidylcholine (DMPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1,2-diarachidoyl-sn-glycero-3-phosphocholine (DBPC), 1-stearoyl-2-palmitoylphosphatidylcholine (SPPC), 1,2-eicosenoyl-sn-glycero-3-phosphocholine (DEPC), lysophosphatidylcholine, dilinoleoylphosphatidylcholine distearoylphosphatidylethanolamine (DSPE), and lysophosphatidylethanolamine.

10. The lipid nanoparticle according to claim 8, wherein the molar ratio of the compound according to any one of claims 1-6 to the helper lipid to the polyethylene glycol lipid is (20-80):(20-80):(0.5-20); preferably, the molar ratio of the compound according to any one of claims 1-6 to the helper lipid to the polyethylene glycol lipid is (30-80):(30-80):(0.5-20).

11. The lipid nanoparticle according to claim 7, wherein the lipid nanoparticle has a diameter of 15-300 nm.

12. Use of the compound according to claims 1-6 and/or the lipid nanoparticle according to claims 7-11 in preparing a bioactive substance delivery system.

13. The use according to claim 12, wherein the bioactive substance is a DNA or an RNA, and the RNA is selected from an antisense RNA, an saRNA, an mRNA, an lncRNA, an miRNA, an siRNA, a piRNA, a gRNA, a tsRNA, a circRNA, and a self-amplifying mRNA;
preferably, the bioactive substance delivery system is an mRNA vaccine;
preferably, the mRNA vaccine is a vaccine for preventing a cancer, a virus infection, a bacterial infection, or a fungal infection;
preferably, the virus is selected from norovirus, Ebola virus, coronavirus, cytomegalovirus, Dengue virus, Zika virus, coxsackievirus, enterovirus, hepatitis virus, herpes simplex virus, human papilloma virus, influenza virus, Marburg virus, measles virus, poliovirus, rabies virus, rotavirus, and/or measles virus.

14. The lipid nanoparticle according to any one of claims 7-11, further comprising a bioactive substance.

15. The lipid nanoparticle according to claim 14, wherein the bioactive substance is a DNA or an RNA, and the RNA is preferably selected from an antisense RNA, an saRNA, an mRNA, an lncRNA, an miRNA, an siRNA, a piRNA, a gRNA, a tsRNA, a circRNA, and a self-amplifying mRNA.

16. The lipid nanoparticle according to claim 14 or 15, wherein a nitrogen-to-phosphorus ratio of the lipid nanoparticle is (1-15):1.

17. A medicament, comprising the lipid nanoparticle according to any one of claims 14-16 and a pharmaceutically acceptable excipient.

18. The medicament according to claim 17, wherein the medicament is a liquid formulation or a lyophilized powder formulation.

19. The medicament according to claim 17, wherein the medicament is a formulation for oral administration, intramuscular injection, subcutaneous injection, intravenous injection, aerosol inhalation, and/or dry powder inhalation.
